# EUROPEAN PATENT APPLICATION

(11) **EP 4 410 839 A1**
(43) Date of publication of application: **07.08.2024**
(21) Application number: 22875046.9
(22) Date of filing: 29.09.2022
(51) Int. Cl.: C07K 16/30, A61K 39/395, A61P 35/00

(54) **ANTI-B7-H3 ANTIBODY AND APPLICATION THEREOF**

(30) Priority: 30.09.2021 CN 202111158382
(71) Applicant: Bio-Thera Solutions, Ltd., Guangzhou, Guangdong 510530 (CN)
(72) Inventor: SU, Huafei, Guangzhou, Guangdong 510530 (CN); ZHONG, Yuting, Guangzhou, Guangdong 510530 (CN); LIANG, Jinchan, Guangzhou, Guangdong 510530 (CN); LIANG, Shide, Guangzhou, Guangdong 510530 (CN); YU, Jin-chen, Guangzhou, Guangdong 510530 (CN); LI, Shengfeng, Guangzhou, Guangdong 510530 (CN)
(74) Representative: Engelhard, Markus
(86) International application number: PCT/CN2022/122444
(87) International publication number: WO 2023/051663

(57) **Abstract**

The present invention provides an anti-B7-H3 antibody and application thereof. The anti-B7-H3 antibody or an antigen-binding fragment can specifically bind to B7-H3, has high affinity and strong internalization capability, and can be used for the diagnosis and/or treatment of tumors (including cancers and benign tumors).

## Description

### TECHNICAL FIELD

The present invention belongs to the field of bio-pharmaceuticals, and particularly relates to an anti-B7-H3 antibody and application thereof.

### BACKGROUND

B7-H3 (B7 homolog 3 protein), also known as CD276, belongs to the type I transmembrane glycoprotein of the B7 ligand family.

Two forms of B7-H3 have now been found: 2Ig-B7-H3 and 4Ig-B7-H3. 2Ig-B7-H3 is expressed in mouse and human cells and it has an extracellular IgV-IgC structure; 4Ig-B7-H3 is expressed only in human cells and consisting of the structure IgV-IgC-IgV-IgC that is in a tandem repeat form. The main existing form of human B7-H3 is 4Ig-B7-H3.

B7-H3 has limited expression levels in normal tissues, but is abnormally high expressed in a variety of human advanced solid tumors, including but not limited to head and neck cancer, kidney cancer, prostate cancer, lung cancer, breast cancer, gastric cancer and liver cancer. Its overexpression is often associated with a relatively poor prognosis and clinical outcome for the patient. Therefore, B7-H3 is considered a diagnostic marker of certain tumors and can be used as an effective target for developing anti-tumor drugs.

### SUMMARY

The present invention provides an anti-B7-H3 antibody or an antigen-binding fragment can specifically bind to B7-H3. In some embodiments, the anti-B7-H3 antibody or antigen-binding fragment has high affinity and strong internalization capability.

Some embodiments provides an antibody or an antigen-binding fragment specifically binds to B7-H3 and comprises one or more of amino acid sequences of (a)-(f):
(a) a VH CDR1 comprising or consisting of an amino acid sequence set forth in SEQ ID NO: 6 or an amino acid sequence having substitutions, deletions or insertions at one or more sites compared with the amino acid sequence set forth in SEQ ID NO: 6;
(b) a VH CDR2 comprising or consisting of an amino acid sequence set forth in SEQ ID NO: 7 or an amino acid sequence having substitutions, deletions or insertions at one or more sites compared with the amino acid sequence set forth in SEQ ID NO: 7;
(c) a VH CDR3 comprising or consisting of an amino acid sequence set forth in SEQ ID NO: 8 or an amino acid sequence having substitutions, deletions or insertions at one or more sites compared with the amino acid sequence set forth in SEQ ID NO: 8;
(d) a VL CDR1 comprising or consisting of an amino acid sequence set forth in any one of SEQ ID NOs: 9-13 or an amino acid sequence having substitutions, deletions or insertions at one or more sites compared with the amino acid sequence set forth in any one of SEQ ID NOs: 9-13;
(e) a VL CDR2 comprising or consisting of an amino acid sequence set forth in any one of SEQ ID NOs: 14-18 or an amino acid sequence having substitutions, deletions or insertions at one or more sites compared with the amino acid sequence set forth in any one of SEQ ID NOs: 14-18; and
(f) a VL CDR3 comprising or consisting of an amino acid sequence set forth in any one of SEQ ID NOs: 19-23 or an amino acid sequence having substitutions, deletions or insertions at one or more sites compared with the amino acid sequence set forth in any one of SEQ ID NOs: 19-23. In some embodiments, the antibody or the antigen-binding fragment specifically binds to B7-H3 and comprises:
   (a) a VH CDR1 comprising or consisting of an amino acid sequence set forth in SEQ ID NO: 6 or an amino acid sequence having substitutions, deletions or insertions at one or more sites compared with the amino acid sequence set forth in SEQ ID NO: 6; and
   (b) a VH CDR2 comprising or consisting of an amino acid sequence set forth in SEQ ID NO: 7 or an amino acid sequence having substitutions, deletions or insertions at one or more sites compared with the amino acid sequence set forth in SEQ ID NO: 7; and
   (c) a VH CDR3 comprising or consisting of an amino acid sequence set forth in SEQ ID NO: 8 or an amino acid sequence having substitutions, deletions or insertions at one or more sites compared with the amino acid sequence set forth in SEQ ID NO: 8.

In some embodiments, the antibody or the antigen-binding fragment comprises a VH CDR1 set forth in SEQ ID NO: 6, a VH CDR2 set forth in SEQ ID NO: 7 and a VH CDR3 set forth in SEQ ID NO: 8.

In some embodiments, the antibody or the antigen-binding fragment specifically binds to B7-H3 and comprises:
(d) a VL CDR1 comprising or consisting of an amino acid sequence set forth in any one of SEQ ID NOs: 9-13 or an amino acid sequence having substitutions, deletions or insertions at one or more sites compared with the amino acid sequence set forth in any one of SEQ ID NOs: 9-13; and
(e) a VL CDR2 comprising or consisting of an amino acid sequence set forth in any one of SEQ ID NOs: 14-18 or an amino acid sequence having substitutions, deletions or insertions at one or more sites compared with the amino acid sequence set forth in any one of SEQ ID NOs: 14-18; and
(f) a VL CDR3 comprising or consisting of an amino acid sequence set forth in any one of SEQ ID NOs: 19-23 or an amino acid sequence having substitutions, deletions or insertions at one or more sites compared with the amino acid sequence set forth in any one of SEQ ID NOs: 19-23.

In some embodiments, the antibody or the antigen-binding fragment comprises a VL CDR1 set forth in SEQ ID NO: 9, a VL CDR2 set forth in SEQ ID NO: 14 and a VL CDR3 set forth in SEQ ID NO: 19.

In some embodiments, the antibody or the antigen-binding fragment comprises a VL CDR1 set forth in SEQ ID NO: 10, a VL CDR2 set forth in SEQ ID NO: 15 and a VL CDR3 set forth in SEQ ID NO: 20.

In some embodiments, the antibody or the antigen-binding fragment comprises a VL CDR1 set forth in SEQ ID NO: 11, a VL CDR2 set forth in SEQ ID NO: 16 and a VL CDR3 set forth in SEQ ID NO: 21.

In some embodiments, the antibody or the antigen-binding fragment comprises a VL CDR1 set forth in SEQ ID NO: 12, a VL CDR2 set forth in SEQ ID NO: 17 and a VL CDR3 set forth in SEQ ID NO: 22.

In some embodiments, the antibody or the antigen-binding fragment comprises a VL CDR1 set forth in SEQ ID NO: 13, a VL CDR2 set forth in SEQ ID NO: 18 and a VL CDR3 set forth in SEQ ID NO: 23.

In some embodiments, the antibody or the antigen-binding fragment specifically binds to B7-H3 and comprises:
(a) a VH CDR1 comprising or consisting of an amino acid sequence set forth in SEQ ID NO: 6 or an amino acid sequence having substitutions, deletions or insertions at one or more sites compared with the amino acid sequence set forth in SEQ ID NO: 6; and
(b) a VH CDR2 comprising or consisting of an amino acid sequence set forth in SEQ ID NO: 7 or an amino acid sequence having substitutions, deletions or insertions at one or more sites compared with the amino acid sequence set forth in SEQ ID NO: 7; and
(c) a VH CDR3 comprising or consisting of an amino acid sequence set forth in SEQ ID NO: 8 or an amino acid sequence having substitutions, deletions or insertions at one or more sites compared with the amino acid sequence set forth in SEQ ID NO: 8; and
(d) a VL CDR1 comprising or consisting of an amino acid sequence set forth in any one of SEQ ID NOs: 9-13 or an amino acid sequence having substitutions, deletions or insertions at one or more sites compared with the amino acid sequence set forth in any one of SEQ ID NOs: 9-13; and
(e) a VL CDR2 comprising or consisting of an amino acid sequence set forth in any one of SEQ ID NOs: 14-18 or an amino acid sequence having substitutions, deletions or insertions at one or more sites compared with the amino acid sequence set forth in any one of SEQ ID NOs: 14-18; and
(f) a VL CDR3 comprising or consisting of an amino acid sequence set forth in any one of SEQ ID NOs: 19-23 or an amino acid sequence having substitutions, deletions or insertions at one or more sites compared with the amino acid sequence set forth in any one of SEQ ID NOs: 19-23. In some embodiments, the substitution is a conservative amino acid substitution.

In some embodiments, the antibody or the antigen-binding fragment specifically binds to B7-H3 and comprises:
(a) a VH CDR1 comprising an amino acid sequence set forth in SEQ ID NO: 6; and
(b) a VH CDR2 comprising an amino acid sequence set forth in SEQ ID NO: 7; and
(c) a VH CDR3 comprising an amino acid sequence set forth in SEQ ID NO: 8; and
(d) a VL CDR1 comprising an amino acid sequence set forth in any one of SEQ ID NOs: 9-13; and
(e) a VL CDR2 comprising an amino acid sequence set forth in any one of SEQ ID NOs: 14-18; and
(f) a VL CDR3 comprising an amino acid sequence set forth in any one of SEQ ID NOs: 19-23.

In some embodiments, the antibody or the antigen-binding fragment comprises a VH CDR1 set forth in SEQ ID NO: 6, a VH CDR2 set forth in SEQ ID NO: 7, a VH CDR3 set forth in SEQ ID NO: 8, a VL CDR1 set forth in SEQ ID NO: 9, a VL CDR2 set forth in SEQ ID NO: 14 and a VL CDR3 set forth in SEQ ID NO: 19.

In some embodiments, the antibody or the antigen-binding fragment comprises a VH CDR1 set forth in SEQ ID NO: 6, a VH CDR2 set forth in SEQ ID NO: 7, a VH CDR3 set forth in SEQ ID NO: 8, a VL CDR1 set forth in SEQ ID NO: 10, a VL CDR2 set forth in SEQ ID NO: 15 and a VL CDR3 set forth in SEQ ID NO: 20.

In some embodiments, the antibody or the antigen-binding fragment comprises a VH CDR1 set forth in SEQ ID NO: 6, a VH CDR2 set forth in SEQ ID NO: 7, a VH CDR3 set forth in SEQ ID NO: 8, a VL CDR1 set forth in SEQ ID NO: 11, a VL CDR2 set forth in SEQ ID NO: 16 and a VL CDR3 set forth in SEQ ID NO: 21.

In some embodiments, the antibody or the antigen-binding fragment comprises a VH CDR1 set forth in SEQ ID NO: 6, a VH CDR2 set forth in SEQ ID NO: 7, a VH CDR3 set forth in SEQ ID NO: 8, a VL CDR1 set forth in SEQ ID NO: 12, a VL CDR2 set forth in SEQ ID NO: 17 and a VL CDR3 set forth in SEQ ID NO: 22.

In some embodiments, the antibody or the antigen-binding fragment comprises a VH CDR1 set forth in SEQ ID NO: 6, a VH CDR2 set forth in SEQ ID NO: 7, a VH CDR3 set forth in SEQ ID NO: 8, a VL CDR1 set forth in SEQ ID NO: 13, a VL CDR2 set forth in SEQ ID NO: 18 and a VL CDR3 set forth in SEQ ID NO: 23.

### Amino acid sequences of VH CDRs (Kabat numbering)

| Type | Amino acid sequence | SEQ ID NO: |
|---|---|---|
| VH CDR1 | DYDIN | 6 |
| VH CDR2 | WIFPGDDTTKYNEKFKG | 7 |
| VH CDR3 | SPSFDY | 8 |

### Amino acid sequences of VL CDRs (Kabat numbering)

| Type | Amino acid sequence | SEQ ID NO: |
|---|---|---|
| VL CDR1 | SASSTIGFMY | 9 |
| | KASQDINRFLS | 10 |
| | RASKSVSTSGYSYMH | 11 |
| | RATGNIHNYLA | 12 |
| | RASKNIYSFLG | 13 |
| VL CDR2 | DTSKLAS | 14 |
| | RANRLRD | 15 |
| | LVSNLES | 16 |
| | SAKTLAD | 17 |
| | NAKTLAE | 18 |
| VL CDR3 | HQRSSYPT | 19 |
| | LQYDEFPPFT | 20 |
| | QHIRELT | 21 |
| | QHFWSIPWT | 22 |
| | QHHYGTPPYT | 23 |

In some embodiments, the antibody or the antigen-binding fragment further comprises a heavy chain constant region, a light chain constant region, an Fc region or a combination thereof. In some embodiments, the light chain constant region is a κ or λ chain constant region. In some embodiments, the antibody or the antigen-binding fragment is of one of the isotypes IgG, IgM, IgA, IgE or IgD. In some embodiments, the isotype is IgG1, IgG2, IgG3 or IgG4. In some embodiments, the antibody or the antigen-binding fragment is a murine antibody, a chimeric antibody, a humanized antibody or a fully human antibody.

In some embodiments, the Fc is a variant Fc region. In some embodiments, the variant Fc region has one or more amino acid modifications, such as substitutions, deletions or insertions, relative to a parent Fc region. In some embodiments, the amino acid modification of the Fc region alters effector function activity relative to the activity of the parent Fc region. In some embodiments, the variant Fc region can have altered (i.e., increased or decreased) antibody-dependent cellular cytotoxicity (ADCC), complement-dependent cytotoxicity (CDC), phagocytosis, opsonization or cell binding. In some embodiments, the amino acid modification of the Fc region can alter the affinity of the variant Fc region for an FcyR (Fcy receptor) relative to the parent Fc region. In some embodiments, the Fc region is derived from IgG1 or IgG4. In some embodiments, the Fc region mutation is N297A, L234A or L235A (Eu numbering). In some embodiments, the Fc region mutation is E345R or S440Y (Eu numbering).

In some embodiments, the antibody or the antigen-binding fragment is an scFV, a Fab, a Fab' or a F(ab)₂. In some embodiments, the antibody or the antigen-binding fragment is a monoclonal antibody.

In some embodiments, a heavy chain variable region of the antibody or the antigen-binding fragment comprises an amino acid sequence set forth in any one of SEQ ID NOs: 24 and 35-38, or an amino acid sequence having at least 80% or 90% identity compared with the amino acid sequence set forth in any one of SEQ ID NOs: 24 and 35-38, or an amino acid sequence having one or more conservative amino acid substitutions compared with the amino acid sequence set forth in any one of SEQ ID NOs: 24 and 35-38; and/or
a light chain variable region of the antibody or the antigen-binding fragment comprises an amino acid sequence set forth in any one of SEQ ID NOs: 25-29 and 39-41, or an amino acid sequence having at least 80% or 90% identity compared with the amino acid sequence set forth in any one of SEQ ID NOs: 25-29 and 39-41, or an amino acid sequence having one or more conservative amino acid substitutions compared with the amino acid sequence set forth in any one of SEQ ID NOs: 25-29 and 39-41.

In some embodiments, a heavy chain variable region of the antibody or the antigen-binding fragment comprises an amino acid sequence set forth in SEQ ID NO: 24, or an amino acid sequence having at least 80% or 90% identity compared with the amino acid sequence set forth in SEQ ID NO: 24, or an amino acid sequence having one or more conservative amino acid substitutions compared with the amino acid sequence set forth in SEQ ID NO: 24; and/or
a light chain variable region of the antibody or the antigen-binding fragment comprises an amino acid sequence set forth in any one of SEQ ID NOs: 25-29, or an amino acid sequence having at least 80% or 90% identity compared with the amino acid sequence set forth in any one of SEQ ID NOs: 25-29, or an amino acid sequence having one or more conservative amino acid substitutions compared with the amino acid sequence set forth in any one of SEQ ID NOs: 25-29.

In some embodiments, a heavy chain variable region of the antibody or the antigen-binding fragment comprises an amino acid sequence set forth in SEQ ID NO: 24, and a light chain variable region of the antibody or the antigen-binding fragment comprises an amino acid sequence set forth in SEQ ID NO: 25.

In some embodiments, a heavy chain variable region of the antibody or the antigen-binding fragment comprises an amino acid sequence set forth in SEQ ID NO: 24, and a light chain variable region of the antibody or the antigen-binding fragment comprises an amino acid sequence set forth in SEQ ID NO: 26.

In some embodiments, a heavy chain variable region of the antibody or the antigen-binding fragment comprises an amino acid sequence set forth in SEQ ID NO: 24, and a light chain variable region of the antibody or the antigen-binding fragment comprises an amino acid sequence set forth in SEQ ID NO: 27.

In some embodiments, a heavy chain variable region of the antibody or the antigen-binding fragment comprises an amino acid sequence set forth in SEQ ID NO: 24, and a light chain variable region of the antibody or the antigen-binding fragment comprises an amino acid sequence set forth in SEQ ID NO: 28.

In some embodiments, a heavy chain variable region of the antibody or the antigen-binding fragment comprises an amino acid sequence set forth in SEQ ID NO: 24, and a light chain variable region of the antibody or the antigen-binding fragment comprises an amino acid sequence set forth in SEQ ID NO: 29.

In some embodiments, a heavy chain variable region of the antibody or the antigen-binding fragment comprises an amino acid sequence set forth in any one of SEQ ID NOs: 35-38, or an amino acid sequence having at least 80% or 90% identity compared with the amino acid sequence set forth in any one of SEQ ID NOs: 35-38, or an amino acid sequence having one or more conservative amino acid substitutions compared with the amino acid sequence set forth in any one of SEQ ID NOs: 35-38; and/or
a light chain variable region of the antibody or the antigen-binding fragment comprises an amino acid sequence set forth in any one of SEQ ID NOs: 39-41, or an amino acid sequence having at least 80% or 90% identity compared with the amino acid sequence set forth in any one of SEQ ID NOs: 39-41, or an amino acid sequence having one or more conservative amino acid substitutions compared with the amino acid sequence set forth in any one of SEQ ID NOs: 39-41.

In some embodiments, a heavy chain variable region of the antibody or the antigen-binding fragment comprises an amino acid sequence set forth in SEQ ID NO: 35, and a light chain variable region of the antibody or the antigen-binding fragment comprises an amino acid sequence set forth in SEQ ID NO: 39.

In some embodiments, a heavy chain variable region of the antibody or the antigen-binding fragment comprises an amino acid sequence set forth in SEQ ID NO: 36, and a light chain variable region of the antibody or the antigen-binding fragment comprises an amino acid sequence set forth in SEQ ID NO: 39.

In some embodiments, a heavy chain variable region of the antibody or the antigen-binding fragment comprises an amino acid sequence set forth in SEQ ID NO: 36, and a light chain variable region of the antibody or the antigen-binding fragment comprises an amino acid sequence set forth in SEQ ID NO: 40.

In some embodiments, a heavy chain variable region of the antibody or the antigen-binding fragment comprises an amino acid sequence set forth in SEQ ID NO: 37, and a light chain variable region of the antibody or the antigen-binding fragment comprises an amino acid sequence set forth in SEQ ID NO: 41.

In some embodiments, a heavy chain variable region of the antibody or the antigen-binding fragment comprises an amino acid sequence set forth in SEQ ID NO: 38, and a light chain variable region of the antibody or the antigen-binding fragment comprises an amino acid sequence set forth in SEQ ID NO: 41.

### Amino acid sequences of variable regions (Kabat numbering)

| Type | Amino acid sequence | SEQ ID NO: |
|---|---|---|
| VH | QVQLQQSGPELVKPGASVRISCKASGYTFTDYDINWVQQRPGQGLEWI GWIFPGDDTTKYNEKFKGRATLTADKSSNTAYMQLNGLTSENSAVYF CARSPSFDYWGQGTLVTVSS | 24 |
| | EVQLVQSGAEVKKSGESLKISCKASGYTFTDYDINWVRQMPGKGLEW MGWIFPGDDTTKYNEKFKGQVTISADKSTNTAYLQWSSLKASDTAM YYCARSPSFDYWGQGTLVTVSS | 35 |
| | EVQLVQSGAEVKKSGESLKISCKASGYTFTDYDINWVRQMPGKGLEWI GWIFPGDDTTKYNEKFKGQVTLSADKSTNTAYMQWSSLKASDTAMY YCARSPSFDYWGQGTLVTVSS | 36 |
| | EVQLVESGGGLVQPGGSLRLSCAASGYTFTDYDINWVRQAPGKGLEW VGWIFPGDDTTKYNEKFKGRFTISADKSKNTAYLQMNSLRAEDTAVY YCARSPSFDYWGQGTLVTVSS | 37 |
| | QVQLVQSGAEVKKPGASVKVSCKASGYTFTDYDINWVRQAPGQRLE WIGWIFPGDDTTKYNEKFKGRATLTADKSASTAYMELSSLRSEDTAVY YCARSPSFDYWGQGTLVTVSS | 38 |
| VL | QIVLTQSPAIMSASPGERVTMTCSASSTIGFMYWYQQKPGTSPKRWIYD TSKLASGVPARFSVSGSGTSYSLTISSMEAEDAATYYCHQRSSYPTFGG GTKLEIK | 25 |
| | DIKMTQSPSSMYASLGERVTITCKASQDINRFLSWFQQKPGKSPKTLIY RANRLRDGVPSRFSGNGSGQDYSLTISSLEYEDMGIYYCLQYDEFPPF TFGSGTKLEIK | 26 |
| | DIVLTQSPASLAVSLGQRATISCRASKSVSTSGYSYMHWNQQKPGQPPR LLIYLVSNLESGVPARFSGSGSGTDFTLNIHPVEEEDAATYYCQHIRELT FGAGTKLELK | 27 |
| | DIQMTQSTASLSAFVGETVTITCRATGNIHNYLAWYHQKQGKSPHLLV YSAKTLADGVPSRFSGSGSGTQYSLQINSLHPEDFGTYYCQHFWSIPW TFGGGTKLEIK | 28 |
| | DIQMTQSPASLSASVGETVTITCRASKNIYSFLGWIQQRQGKSPQSLVY NAKTLAEGVPSRFSGSGSGTQFSLKINSLQPEDFGNYYCQHHYGTPPY TFGGGTKLEIK | 29 |
| | [?] QIVLTQSPGTLSLSPGERATLSCSASSTIGFMYWYQQKPGQAPRRWIYD TSKLASGVPDRFSVSGSGTDYTLTISRLEPEDFAVYYCHQRSSYPTFGQ GTKVEIK | 39 |
| | QIVLTQSPGTLSLSPGERATLSCSASSTIGFMYWYQQKPGQAPRRWIYD TSKLASGVPDRFSGSGSGTDYTLTISRLEPEDFAVYYCHQRSSYPTFGQ GTKVEIK | 40 |
| | QIVLTQSPSSLSASVGDRVTITCSASSTIGFMYWYQQKPGKAPKRWIYD TSKLASGVPSRFSVSGSGTDYTLTISSLQPEDFATYYCHQRSSYPTFGQ GTKVEIK | 41 |

In some embodiments, a heavy chain constant region of the antibody or the antigen-binding fragment comprises an amino acid sequence set forth in SEQ ID NO: 32 or 33, or an amino acid sequence having at least 80% or 90% identity compared with the amino acid sequence set forth in SEQ ID NO: 32 or 33, or an amino acid sequence having one or more conservative amino acid substitutions compared with the amino acid sequence set forth in SEQ ID NO: 32 or 33; and/or
a light chain constant region of the antibody or the antigen-binding fragment comprises an amino acid sequence set forth in SEQ ID NO: 34, or an amino acid sequence having at least 80% or at least 90% identity compared with the amino acid sequence set forth in SEQ ID NO: 34, or an amino acid sequence having one or more conservative amino acid substitutions compared with the amino acid sequence set forth in SEQ ID NO: 34.

In some embodiments, provided is an antibody that specifically binds to B7-H3, a heavy chain of the antibody comprises a heavy chain variable region having an amino acid sequence set forth in SEQ ID NO: 24 and a heavy chain constant region having an amino acid sequence set forth in SEQ ID NO: 32; a light chain of the antibody comprises a light chain variable region having an amino acid sequence set forth in SEQ ID NO: 25 and a light chain constant region having an amino acid sequence set forth in SEQ ID NO: 34.

In some embodiments, a heavy chain of the antibody comprises a heavy chain variable region having an amino acid sequence set forth in SEQ ID NO: 24 and a heavy chain constant region having an amino acid sequence set forth in SEQ ID NO: 32; a light chain of the antibody comprises a light chain variable region having an amino acid sequence set forth in SEQ ID NO: 26 and a light chain constant region having an amino acid sequence set forth in SEQ ID NO: 34.

In some embodiments, a heavy chain of the antibody comprises a heavy chain variable region having an amino acid sequence set forth in SEQ ID NO: 24 and a heavy chain constant region having an amino acid sequence set forth in SEQ ID NO: 32; a light chain of the antibody comprises a light chain variable region having an amino acid sequence set forth in SEQ ID NO: 27 and a light chain constant region having an amino acid sequence set forth in SEQ ID NO: 34.

In some embodiments, a heavy chain of the antibody comprises a heavy chain variable region having an amino acid sequence set forth in SEQ ID NO: 24 and a heavy chain constant region having an amino acid sequence set forth in SEQ ID NO: 32; a light chain of the antibody comprises a light chain variable region having an amino acid sequence set forth in SEQ ID NO: 28 and a light chain constant region having an amino acid sequence set forth in SEQ ID NO: 34.

In some embodiments, a heavy chain of the antibody comprises a heavy chain variable region having an amino acid sequence set forth in SEQ ID NO: 24 and a heavy chain constant region having an amino acid sequence set forth in SEQ ID NO: 32; a light chain of the antibody comprises a light chain variable region having an amino acid sequence set forth in SEQ ID NO: 29 and a light chain constant region having an amino acid sequence set forth in SEQ ID NO: 34.

In some embodiments, a heavy chain of the antibody comprises a heavy chain variable region having an amino acid sequence set forth in SEQ ID NO: 35 and a heavy chain constant region having an amino acid sequence set forth in SEQ ID NO: 32; a light chain of the antibody comprises a light chain variable region having an amino acid sequence set forth in SEQ ID NO: 39 and a light chain constant region having an amino acid sequence set forth in SEQ ID NO: 34.

In some embodiments, a heavy chain of the antibody comprises a heavy chain variable region having an amino acid sequence set forth in SEQ ID NO: 36 and a heavy chain constant region having an amino acid sequence set forth in SEQ ID NO: 32; a light chain of the antibody comprises a light chain variable region having an amino acid sequence set forth in SEQ ID NO: 39 and a light chain constant region having an amino acid sequence set forth in SEQ ID NO: 34.

In some embodiments, a heavy chain of the antibody comprises a heavy chain variable region having an amino acid sequence set forth in SEQ ID NO: 36 and a heavy chain constant region having an amino acid sequence set forth in SEQ ID NO: 32; a light chain of the antibody comprises a light chain variable region having an amino acid sequence set forth in SEQ ID NO: 40 and a light chain constant region having an amino acid sequence set forth in SEQ ID NO: 34.

In some embodiments, a heavy chain of the antibody comprises a heavy chain variable region having an amino acid sequence set forth in SEQ ID NO: 37 and a heavy chain constant region having an amino acid sequence set forth in SEQ ID NO: 32; a light chain of the antibody comprises a light chain variable region having an amino acid sequence set forth in SEQ ID NO: 41 and a light chain constant region having an amino acid sequence set forth in SEQ ID NO: 34.

In some embodiments, a heavy chain of the antibody comprises a heavy chain variable region having an amino acid sequence set forth in SEQ ID NO: 38 and a heavy chain constant region having an amino acid sequence set forth in SEQ ID NO: 32; a light chain of the antibody comprises a light chain variable region having an amino acid sequence set forth in SEQ ID NO: 41 and a light chain constant region having an amino acid sequence set forth in SEQ ID NO: 34.

### Amino acid sequences of constant regions

| Name | Amino acid sequence | SEQ ID NO: |
|---|---|---|
| IgG1 heavy chain constant region | | 32 |
| IgG4 heavy chain constant region | ASTKGPSVFPLAPCSRSTSESTAALGCLVKDYFPEPVTVSWNSGAL TSGVHTFPAVLQSSGLYSLSSVVTVPSSSLGTKTYTCNVDHKPSNT KVDKRVESKYGPPCPPCPAPEFLGGPSVFLFPPKPKDTLMISRTPEV TCVVVDVSQEDPEVQFNWYVDGVEVHNAKTKPREEQFNSTYRV VSVLTVLHQDWLNGKEYKCKVSNKGLPSSIEKTISKAKGQPREPQ VYTLPPSQEEMTKNQVSLTCLVKGFYPSDIAVEWESNGQPENNYK TTPPVLDSDGSFFLYSRLTVDKSRWQEGNVFSCSVMHEALHNHYT QKSLSLSLGK | 33 |
| Light chain constant region | | 34 |

In some embodiments, a heavy chain of the antibody comprises an amino acid sequence set forth in SEQ ID NO: 50; a light chain of the antibody comprises an amino acid sequence set forth in SEQ ID NO: 52.

In some embodiments, the antibody or the antigen-binding fragment is a monoclonal antibody (including a full-length monoclonal antibody) or a multispecific antibody or an antigen-binding fragment (e.g., a bispecific antibody or an antigen-binding fragment).

In some embodiments, the antibody has two heavy chains with the same sequence and two light chains with the same sequence. In some embodiments, the Fc regions pair to form disulfide bonds. In some embodiments, the antibody or the antigen-binding fragment is an isolated antibody or an antigen-binding fragment.

The present invention further provides a polynucleotide encoding the antibody or the antigen-binding fragment. In some embodiments, the polynucleotide is an isolated polynucleotide. In some embodiments, the polynucleotide sequence is selected from the nucleic acid sequences set forth in SEQ ID NO: 49 and 51.

The present invention further provides an expression vector comprising the polynucleotide. In some embodiments, the expression vector comprising the polynucleotide is a nucleic acid fragment, a plasmid, a phage, or a virus. In some embodiments, the expression vector is an isolated expression vector.

The present invention further provides a host cell comprising the polynucleotide or the expression vector. In some embodiments, the host cell is an isolated host cell. In some embodiments, the host cell is a CHO cell, a HEK293 cell (e.g., a HEK293F cell), a BHK cell, a Cos1 cell, a Cos7 cell, a CV1 cell, or a murine L cell.

The present invention further provides a pharmaceutical composition comprising the antibody or the antigen-binding fragment or the biomaterial, and a pharmaceutically acceptable carrier.

The present invention further provides use of the antibody or the antigen-binding fragment, the biomaterial, or the pharmaceutical composition in the manufacture of a drug for diagnosing and/or treating a disease. In some embodiments, the present invention provides use of the antibody or the antigen-binding fragment, the biomaterial, or the pharmaceutical composition in the diagnosis and/or treatment of a disease. In some embodiments, the present invention provides a method for diagnosing and/or treating a disease, wherein the method comprises administering to a patient in need thereof an effective dose of the antibody or the antigen-binding fragment, the biomaterial, or the pharmaceutical composition.

In some embodiments, the drug is one that inhibits the activity of B7-H3. In some embodiments, the disease is selected from a tumor, a respiratory disease, a skin and musculoskeletal disease, a genitourinary disease, a nervous system disease and a digestive system disease. In some embodiments, the tumor is a benign tumor or cancer. In some embodiments, the tumor is a hematological tumor or a solid tumor. In some embodiments, the disease includes a tumor positive for B7-H3 expression. In some embodiments, the tumor is selected from melanoma, lung cancer (e.g., small cell lung cancer and non-small cell lung cancer), breast cancer (e.g., triple-negative breast cancer), ovarian cancer (e.g., ovarian epithelial carcinoma), glioma (e.g., glioblastoma and childhood brain stem glioma), prostate cancer (e.g., castration-resistant prostate cancer), pancreatic cancer (e.g., pancreatic ductal carcinoma), head and neck cancer, leukemia (e.g., acute myeloid leukemia (AML)), cervical cancer, kidney cancer, squamous cell tumor, squamous cell carcinoma (e.g., squamous cell lung cancer or head and neck squamous cell carcinoma), colorectal cancer, gastric cancer, liver cancer, mesothelioma, anal cancer, skin cancer, vulval cancer, neuroblastoma, desmoplastic small round cell tumor, medulloblastoma, meningioma, peritoneal malignancy, sarcoma, brain cancer, central nervous system tumor and metastatic brain tumor.

The present invention further provides a diagnostic method and use. In some embodiments, provided is a method for detecting B7-H3 expression in a sample, wherein the method comprises contacting the sample with the antibody or the antigen-binding fragment such that the antibody or the antigen-binding fragment binds to B7-H3, and detecting the binding, i.e., an amount of B7-H3 in the sample. In some embodiments, provided is use of the antibody or the antigen-binding fragment in the manufacture of a kit for the diagnosis or prognosis of a tumor (including benign tumors and cancers). In some embodiments, provided is a diagnostic or prognostic kit comprising the antibody or the antigen-binding fragment.

The present invention provides an anti-B7-H3 antibody or an antigen-binding fragment and use thereof. The anti-B7-H3 antibody or the antigen-binding fragment of the present invention can specifically bind to B7-H3, have high specificity, affinity, and strong internalization capability, and can be used for the development of ADC drugs, with the potential to obtain better anti-tumor activity and efficacy, and can also be used for the diagnosis and prognosis of a tumor (including benign tumors and cancers).

### BRIEF DESCRIPTION OF THE DRAWINGS

FIG. 1 shows the binding capacity of chimeric antibodies to MDA-MB-468 cells, wherein H1L4 is the control antibody M30-H1-L4.
FIG. 2 shows the binding capacity of chimeric antibodies to Lag3-CHO cells, VISTA-CHO cells, Tim3-CHO cells and Raji cells.
FIG. 3 shows the binding capacity of humanized antibodies to hB7-H3-Fc, wherein H1L4 represents the control antibody M30-H1-L4.
FIG. 4 shows the binding capacity of humanized antibodies to MDA-MB-468 cells; wherein H1L4 represents the control antibody M30-H1-L4.
FIG. 5 shows the binding capacity of humanized antibodies to VISTA-CHO cells, Tim3-CHO cells and Lag3-CHO cells, wherein H1L4 represents the control antibody M30-H1-L4.

### DETAILED DESCRIPTION

Unless otherwise defined, all technical and scientific terms used herein have the same meaning as that commonly understood by those of ordinary skill in the art to which the present invention belongs.

### Definitions

It should be noted that the term "an" entity refers to one or more of the entities. For example, "an antibody" should be interpreted as one or more antibodies. As such, the terms "a" (or "an"), "one or more" and "at least one" can be used interchangeably herein.

As used herein, the term "contain" or "comprise" means that the antibodies, compositions, methods or the like comprise the recited elements, such as components or steps and do not exclude the others. "Consisting essentially of..." means that the antibodies, compositions, methods or the like exclude other elements that have a fundamental impact on the characteristics of the combination, but do not exclude elements that do not substantially affect the characteristics of the antibodies, compositions, methods or the like. "Consisting of..." means that elements not specifically listed are excluded.

The term "polypeptide" is intended to encompass both the singular form "polypeptide" and the plural form "polypeptides", and refers to a molecule consisting of amino acid monomers linearly linked by amide bonds (also known as peptide bonds). The term "polypeptide" refers to any single chain or multiple chains of two or more amino acids and does not refer to a specific length of the product. Thus, included within the definition of "polypeptide" are peptides, dipeptides, tripeptides, oligopeptides, "proteins", "amino acid chains" or any other term used to refer to chains of two or more amino acids, and the term "polypeptide" may be used in place of, or interchangeably with, any of the above terms. The term "polypeptide" is also intended to refer to a product of post-expression polypeptide modification, including but not limited to glycosylation, acetylation, phosphorylation, amidation, derivatization by known protecting/blocking groups, proteolytic cleavage, or non-naturally occurring amino acid modification. The polypeptide may be derived from a natural biological source or produced by recombinant techniques, but it is not necessarily translated from a specified nucleic acid sequence. It may be produced in any manner, including chemical synthesis.

"Amino acid" refers to an organic compound containing both an amino group and a carboxyl group, such as an α-amino acid that can be encoded by a nucleic acid, either directly or in the form of a precursor. A single amino acid is encoded by a nucleic acid consisting of three nucleotides (so-called codon or base triplet). Each amino acid is encoded by at least one codon. The encoding of the same amino acid by different codons is known as "codon degeneracy". Amino acids include natural amino acids and non-natural amino acids.

As used herein, the twenty conventional amino acids and their abbreviations follow conventional usage. Stereoisomers (e.g., D-amino acids) of the twenty conventional amino acids, unnatural amino acids (such as α- or α-disubstituted amino acids), N-alkyl amino acids, lactic acid, and other unconventional amino acids can also be suitable components for the polypeptides of the present disclosure. Examples of unconventional amino acids include: 4-hydroxyproline, γ-carboxyglutamate, ε-N,N,N-trimethyllysine, ε-N-acetyllysine, O-phosphoserine, N-acetylserine, N-formylmethionine, 3-methylhistidine, 5-hydroxylysine, σ-N-methylarginine and other similar amino acids and imino acids (e.g., 4-hydroxyproline). In the polypeptide notation used herein, the left-hand direction is the amino-terminal direction and the right-hand direction is the carboxy-terminal direction, in accordance with standard usage and convention. The conventional (natural) amino acids include alanine (three-letter symbol: Ala, one-letter symbol: A), arginine (Arg, R), asparagine (Asn, N), aspartic acid (Asp, D), cysteine (Cys, C), glutamine (Gln, Q), glutamic acid (Glu, E), glycine (Gly, G), histidine (His, H), isoleucine (Ile, I), leucine (Leu, L), lysine (Lys, K), methionine (Met, M), phenylalanine (Phe, F), proline (Pro, P), serine (Ser, S), threonine (Thr, T), tryptophan (Trp, W), tyrosine (Tyr, Y), and valine (Val, V).

Minor variations in the amino acid sequences of antibodies or immunoglobulin molecules are contemplated by the present disclosure, provided that the identity of the amino acid sequences is maintained to be at least 75%, such as at least 80%, 90%, 95%, and as another example 99%. In some embodiments, the variations are conservative amino acid substitutions. Conservative amino acid substitutions are ones that occur within a family of amino acids that are related in their side chains. Genetically encoded amino acids are generally classified into the following categories: (1) acidic amino acids are aspartate and glutamate; (2) basic amino acids are lysine, arginine and histidine; (3) non-polar amino acids are alanine, valine, leucine, isoleucine, proline, phenylalanine, methionine, tryptophan; and (4) uncharged polar amino acids are glycine, asparagine, glutamine, cysteine, serine, threonine and tyrosine. Amino acids of the other families include (i) serine and threonine of the aliphatic-hydroxy family; (ii) asparagine and glutamine of the amide-containing family; (iii) alanine, valine, leucine and isoleucine of the aliphatic family; and (iv) phenylalanine, tryptophan and tyrosine of the aromatic family. In some embodiments, conservative amino acid substitution groups are valine-leucine-isoleucine, phenylalanine-tyrosine, lysine-arginine, alanine-valine, glutamic acid-aspartic acid, and asparagine-glutamine. For example, it is reasonable to expect that the single replacement of leucine with isoleucine or valine, aspartate with glutamate, threonine with serine, or the similar replacement of an amino acid with a structurally related amino acid, will not have a major effect on the binding or properties of the resulting molecule, particularly when the replacement does not involve an amino acid within a binding site. Whether an amino acid change results in a functional peptide can be readily determined by determining the specific activity of the polypeptide derivative. Fragments or analogs of antibodies or immunoglobulin molecules can be readily prepared by those of ordinary skill in the art.

In some embodiments, the amino acid substitutions have the following effects: (1) reducing susceptibility to proteolysis, (2) reducing susceptibility to oxidation, (3) altering the binding affinity for formation of protein complexes, (4) altering binding affinity, and (5) conferring or improving other physicochemical or functional properties of such analogs. Analogs can include various muteins whose sequences differ from the naturally occurring peptide sequences. For example, single or multiple amino acid substitutions (preferably conservative amino acid substitutions) may be made in the naturally occurring sequence (preferably in a portion of the polypeptide outside the domains that form intermolecular contacts). A conservative amino acid substitution should not significantly alter the structural characteristics of the parent sequence (e.g., a replacement amino acid should not tend to disrupt a helix that occurs in the parent sequence, or disrupt other types of secondary structures that characterize the parent sequence).

The number of amino acids of conservative amino acid substitutions of VL or VH is about 1, about 2, about 3, about 4, about 5, about 6, about 8, about 9, about 10, about 11, about 13, about 14, about 15 conservative amino acid substitutions, or a range between any two of these values (inclusive) or any value therein. The number of amino acids of conservative amino acid substitutions of a heavy chain constant region, a light chain constant region, a heavy chain, or a light chain is about 1, about 2, about 3, about 4, about 5, about 6, about 8, about 9, about 10, about 11, about 13, about 14, about 15, about 18, about 19, about 22, about 24, about 25, about 29, about 31, about 35, about 38, about 41, about 45 conservative amino acid substitutions, or a range between any two of these values (inclusive) or any value therein.

The term "isolated" as used herein with respect to cells, nucleic acids, polypeptides, antibodies and the like, e.g., "isolated" DNA, RNA, polypeptides, antibodies, refers to molecules that are separated from one or more other components, e.g., DNA or RNA, in the natural environment of the cell. The term "isolated" as used herein also refers to nucleic acids or peptides that are substantially free of cellular materials, viral materials or cell media when produced by recombinant DNA techniques, or of chemical precursors or other chemicals when chemically synthesized. In addition, "isolated nucleic acid" is intended to include nucleic acid fragments that do not and will not occur in nature. The term "isolated" is also used herein to refer to cells or polypeptides that are separated from other cellular proteins or tissues. Isolated polypeptides are intended to include both purified and recombinant polypeptides. Isolated polypeptides, antibodies and the like are usually prepared by at least one purification step. In some embodiments, the purity of the isolated nucleic acids, polypeptides, antibodies and the like is at least about 50%, about 60%, about 70%, about 80%, about 90%, about 95%, about 99%, or a range between any two of these values (inclusive) or any value therein.

The term "encoding" as applied to a polynucleotide refers to a polynucleotide known to "encode" a polypeptide. When in its native state or manipulated by methods well known to those skilled in the art, the polynucleotide can be transcribed and/or translated to produce the polypeptide and/or a fragment thereof.

The term "recombinant", with regard to a polypeptide or polynucleotide, is intended to refer to a polypeptide or polynucleotide that does not occur in nature, and non-limiting examples can be combined to produce a polynucleotide or polypeptide that does not normally occur.

"Homology", "identity" or "similarity" refers to sequence similarity between two peptides or between two nucleic acid molecules. Homology or identity can be determined by comparing the positions that can be aligned in the sequences. When a position of the compared sequences is occupied by the same base or amino acid, the molecules are homologous or identical at that position. The degree of homology between the sequences is a function of the number of matching or homologous positions shared by the sequences. "At least 80% identity" refers to about 80% identity, about 81% identity, about 82% identity, about 83% identity, about 85% identity, about 86% identity, about 87% identity, about 88% identity, about 90% identity, about 91% identity, about 92% identity, about 94% identity, about 95% identity, about 98% identity, about 99% identity, or a range between any two of these values (inclusive) or any value therein. "At least 90% identity" refers to about 90% identity, about 91% identity, about 92% identity, about 93% identity, about 95% identity, about 96% identity, about 97% identity, about 98% identity, about 99% identity, or a range between any two of these values (inclusive) or any value therein.

A polynucleotide consists of a specific sequence of four bases: adenine (A), cytosine (C), guanine (G) and thymine (T)/uracil (U, instead of thymine when the polynucleotide is RNA). A "polynucleotide sequence" can be a letter representation of a polynucleotide molecule. The letter representation can be input into a database in a computer with a central processing unit and used for bioinformatics applications, such as functional genomics and homology searches.

The terms "polynucleotide" and "oligonucleotide" are used interchangeably to refer to a polymeric form of nucleotides of any length, whether deoxyribonucleotides or ribonucleotides or analogs thereof. The polynucleotide may have any three-dimensional structure and may perform any function, known or unknown. The following are non-limiting examples of polynucleotides: genes or gene fragments (such as probes, primers, EST or SAGE tags), exons, introns, messenger RNA (mRNA), transfer RNA, ribosomal RNA, ribozymes, cDNA, dsRNA, siRNA, miRNA, recombinant polynucleotides, branched polynucleotides, plasmids, vectors, isolated DNA of any sequence, isolated RNA of any sequence, and nucleic acid probes and primers. Polynucleotides can include modified nucleotides, such as methylated nucleotides and nucleotide analogs. If present, structural modifications to the nucleotide can be made before or after the assembly of the polynucleotide. The sequence of nucleotides can be interrupted by non-nucleotide components. The polynucleotide may be further modified after polymerization, such as by conjugation with a labeling component. This term also refers to double-stranded and single-stranded molecules. Unless otherwise stated or required, examples of any polynucleotide disclosed herein include a double-stranded form and each of the two complementary single-stranded forms known or predicted to constitute the double-stranded form.

A nucleic acid or a polynucleotide sequence (or a polypeptide or an antibody sequence) having a certain percentage (e.g., 90%, 95%, 98% or 99%) of "identity or sequence identity" to another sequence refers to that when the sequences are aligned, the percentage of bases (or amino acids) in the compared sequences are the same. This alignment and identity percentage or sequence identity can be determined using visual inspection or software programs known in the art, such as the software programs described in Ausubel et al. eds., (2007), Current Protocols in Molecular Biology*.* Preferably, the alignment is performed using default parameters. One alignment program is BLAST using default parameters, such as BLASTN and BLASTP, both using the following default parameters: Genetic code = standard; filter = none; strand = both; cutoff = 60; expect = 10; Matrix = BLOSUM62; Descriptions = 50 sequences; sort by = HIGH SCORE; Databases = non-redundant; GenBank + EMBL + DDBJ + PDB + GenBank CDS translations + SwissProtein + SPupdate + PIR. Biologically equivalent polynucleotides are polynucleotides having the above-specified percentage identity and encoding polypeptides having identical or similar biological activity.

"Antibody", "antigen-binding fragment" refers to a polypeptide or polypeptide complex that specifically recognizes and binds to an antigen. The antibody may be an intact antibody and any antigen-binding fragment or single chain thereof. The term "antibody" thus includes any protein or peptide comprising, in its molecule, at least a portion of an immunoglobulin molecule that has biological activity for binding to an antigen. The antibody, the antigen-binding fragment include, but are not limited to, complementarity determining regions (CDRs) of a heavy or light chain or a ligand binding portion thereof, heavy chain variable regions (VHs), light chain variable regions (VLs), heavy chain constant regions (CHs), light chain constant regions (CLs), framework regions (FRs) or any portion thereof, or at least a portion of a binding protein. The CDRs include CDRs of light chain variable region (VL CDR1-3) and CDRs of heavy chain variable region (VH CDR1-3). An antibody or an antigen-binding fragment can specifically recognize and bind to polypeptides or polypeptide complexes of one or more (e.g., two) antigens. The antibody or the antigen-binding fragment that specifically recognizes and binds to multiple (e.g., two) antigens can be referred to as a multispecific (e.g., bispecific) antibody or antigen-binding fragment.

The terms "antibody fragment", or "antigen-binding fragment" refers to a part of an antibody, and the composition of the antibody fragment of the present invention may be similar to F(ab')₂, F(ab)₂, Fab', Fab, Fv, scFv and the like in monospecific antibody fragments. Regardless of its structure, the antibody fragment binds to the same antigen recognized by an intact antibody. The term "antibody fragment" includes aptamers, mirror image isomers, and bivalent antibodies. The term "antigen-binding fragment" also includes any synthetic or genetically engineered protein that functions as an antibody by binding to a specific antigen to form a complex.

"Single-chain variable fragment" or "scFv" refers to a fusion protein of a heavy chain variable region (VH) and a light chain variable region (VL) of an immunoglobulin. In some aspects, these regions are linked to a short linker peptide having about 10 to about 25 amino acids. The linker may be enriched with glycine to improve flexibility, and enriched with serine or threonine to improve solubility, and may link the N terminus of VH and the C terminus of VL, or vice versa. Although the protein has the constant region removed and the linker introduced, it retains the specificity of the original immunoglobulin. ScFv molecules are generally known in the art and are described, for example, in U.S. patent No. 5,892,019.

The term "antibody" includes a wide variety of polypeptides that can be biochemically distinguished. Those skilled in the art will appreciate that the classes of heavy chains include gamma, mu, alpha, delta, or epsilon (γ, µ, α, δ, or ε), and some subclasses (e.g., γ1-γ4). The nature of this chain determines the "type" of the antibody as IgG, IgM, IgA, IgG or IgE. Immunoglobulin subclasses (isotypes), such as IgG1, IgG2, IgG3, IgG4, IgG5, etc., have been well characterized and the functional specificity imparted is also known. All types of immunoglobulins are within the scope of the present invention. In some embodiments, the immunoglobulin molecule is of the IgG class. Two heavy chains and two light chains are connected in a "Y" configuration through disulfide bonds, wherein the light chain starts at the opening of "Y" configuration and extends through the variable region to surround the heavy chain.

The antibodies, antigen-binding fragments or derivatives disclosed herein include, but are not limited to, polyclonal antibodies, monoclonal antibodies, multispecific antibodies, fully human antibodies, humanized antibodies, primatized antibodies, chimeric antibodies, single-chain antibodies, epitope-binding fragments (e.g., Fab, Fab' and F(ab')₂), and single chain Fv (scFv). Light chains can be classified into kappa (κ) or lambda (λ). Each heavy chain may bind to a κ or λ light chain. In general, when an immunoglobulin is produced by a hybridoma, a B cell or a genetically engineered host cell, the light and heavy chains are connected by covalent bonds, and the "tail" portions of the two heavy chains are connected by covalent disulfide bonds or non-covalent bonds. In the heavy chains, the amino acid sequence extends from the N terminus at the forked end of the Y configuration to the C terminus at the bottom of each chain. The immunoglobulin κ light chain variable region is V_{κ}; and the immunoglobulin λ light chain variable region is V_{λ}.

The terms "constant" and "variable" are used in accordance with function. The light chain variable region (VL) and the heavy chain variable region (VH) determine the antigen recognition and specificity. The light chain constant region (CL) and the heavy chain constant region (CH) impart important biological properties such as secretion, transplacental movement, Fc receptor binding, complement fixation, etc. By convention, the numbering of amino acids in the constant regions increases as they become further away from the antigen-binding site of the antibody or amino terminus. The N-terminal portion is the variable region, and the C-terminal portion is the constant region; the CH3 and CL domains actually comprise the carboxyl termini of the heavy chain and light chain, respectively.

In naturally occurring antibodies, the six "complementarity determining regions" or "CDRs" present in each antigen-binding domain are short, non-contiguous, antigen-specific binding amino acid sequences that form the antigen-binding domain, assuming that the antibody is present in its three-dimensional configuration in an aqueous environment. The remaining amino acids in the antigen-binding domain, referred to as the "framework" region, exhibit little intermolecular variability. Most of the framework regions adopt a β-sheet conformation, with the CDRs forming a loop structure connected to, or in some cases forming part of the β-sheet structure. Thus, the framework regions position the CDRs in a correct orientation by interchain non-covalent interactions through forming a scaffold. The antigen-binding domain with the specifically positioned CDRs forms a surface complementary to an epitope on the antigen that facilitates non-covalent binding of the antibody to its antigenic epitope. For a given heavy or light chain variable region, amino acids comprising the CDRs and the framework regions may be identified by one of ordinary skill in the art according to known methods.

As used herein, the term "CDR" refers to the complementarity determining regions within an antibody variable region. Three CDRs are present in each of the heavy chain variable region and the light chain variable region and named CDR1, CDR2 and CDR3 (or specifically, VH CDR1, VH CDR2, VH CDR3, VL CDR1, VL CDR2 and VL CDR3) for each variable region. Based on different assignment systems, the boundaries of the CDRs of the variable regions of the same antibody may differ. Thus, when it comes to defining an antibody with a particular CDR sequence defined in the present invention, the scope of the antibody also encompasses antibodies whose variable region sequences compri se the CDR sequence of the present invention but whose claimed CDR boundaries differ from the particular CDR boundaries defined in the present invention due to the application of different schemes. CDRs defined according to Kabat and Chothia schemes include overlaps or subsets of amino acid residues when compared with each other. Nevertheless, it is within the scope of the present invention to apply any definition to refer to the CDRs of an antibody or a variant thereof. The exact number of residues comprising a specific CDR will vary according to the sequence and size of the CDR. Those skilled in the art can generally determine which specific residues a CDR comprises based on the variable region amino acid sequence of an antibody.

As used herein, the term "framework" or "framework sequence" refers to the sequence remaining after the CDRs have been subtracted from the variable region. Because the exact definition of a CDR sequence can be determined by different systems, the meaning of a framework sequence is subject to correspondingly different interpretations. The six CDRs (CDR1, CDR2 and CDR3 of light chain and CDR1, CDR2 and CDR3 of heavy chain) divide the framework regions on the light chain and the heavy chain into four sub-regions (FR1, FR2, FR3 and FR4) on each chain, in which CDR1 is positioned between FR1 and FR2, CDR2 between FR2 and FR3, and CDR3 between FR3 and FR4. Without specifying the particular sub-regions as FR1, FR2, FR3 or FR4, a framework region, as referred to by others, represents the combined FRs within the variable region of a single, naturally occurring immunoglobulin chain. As used herein, a FR represents one of the four sub-regions, and FRs represent two or more of the four sub-regions constituting a framework region.

The framework and CDR regions of a humanized antibody need not correspond precisely to the parental sequences, e.g., the donor antibody CDR or the consensus framework may be mutagenized by substitution, insertion and/or deletion of at least one amino acid residue so that the CDR or framework residue at that site does not correspond to either the donor antibody or the consensus framework. Usually, at least 80%, at least 85%, at least 90% or at least 95% of the humanized antibody residues will correspond to those of the parental FR and CDR sequences. As used herein, the term "consensus framework" refers to the framework region in the consensus immunoglobulin sequence. As used herein, the term "consensus immunoglobulin sequence" refers to the sequence formed from the most frequently occurring amino acids (or nucleotides) in a family of related immunoglobulin sequences. In a family of immunoglobulins, each position in the consensus sequence is occupied by the amino acid occurring most frequently at that position in the family. If two amino acids occur equally frequently, either can be included in the consensus sequence.

Where the terms used and/or accepted in the art have two or more definitions, the definitions of the terms used herein include all of these meanings unless explicitly indicated as opposite. One specific example is the use of the term "complementarity determining regions" ("CDRs") to describe non-contiguous antigen-binding sites found within the variable regions of heavy and light chain polypeptides.

Kabat et al. also define a numbering scheme applicable to the variable region sequence of any antibody. One of ordinary skills in the art can apply the "Kabat numbering" scheme to any variable region sequence without depending on other experimental data beyond the sequence itself. "Kabat numbering" refers to the numbering system proposed by Kabat et al., U.S. Dept. of Health and Human Services in Sequence of Proteins of Immunological Interest (1983). EU or Chothia numbering scheme can also be applicable to the antibody.

The antibodies disclosed herein may be derived from any animal, including fish, birds and mammals. Preferably, the antibody is derived from a human being, a mouse, a donkey, a rabbit, a goat, a camel, a llama, a horse, or a chicken source. In another embodiment, the variable region may be derived from a condricthoid source (e.g., from a shark).

The "heavy chain constant region" comprises at least one of a CH1 domain, a hinge (e.g., upper, middle, and/or lower hinge region) domain, a CH2 domain, a CH3 domain, or a variant or a fragment. The heavy chain constant regions of the antibody may be derived from different immunoglobulin molecules. For example, the heavy chain constant regions of the polypeptide may comprise a CH1 domain derived from an IgG1 molecule and a hinge region derived from an IgG3 molecule. In another embodiment, the heavy chain constant region may comprise a hinge region derived partially from an IgG1 molecule and partially from an IgG3 molecule. In another embodiment, a portion of the heavy chain may comprise a chimeric hinge region derived partially from an IgG1 molecule and partially from an IgG4 molecule.

"Light chain constant region" includes a part of amino acid sequence from the light chain of an antibody. Preferably, the light chain constant region comprises at least one of a constant κ domain or a constant λ domain. "Light chain-heavy chain pair" refers to a collection of light and heavy chains that can form dimers through disulfide bonds between the CL domain of the light chain and the CH1 domain of the heavy chain.

The "VH domain" includes the variable domain at the amino terminus of an immunoglobulin heavy chain. The "CH1 domain" includes the first constant region of an immunoglobulin heavy chain. The CH2 domain is not closely paired with other domains, but rather two N-linked branched carbohydrate chains are inserted between the two CH2 domains of an intact native IgG molecule. The CH3 domain extends from the CH2 domain to the C terminus of the IgG molecule and comprises about 108 residues. "Hinge region" includes a portion of the heavy chain region connecting the CH1 domain and CH2 domain. The hinge region comprises about 25 residues and is flexible, thereby enabling independent movement of the two N-terminal antigen-binding regions. The hinge region can be subdivided into three distinct domains: upper, middle and lower hinge domains.

"Disulfide bond" refers to a covalent bond formed between two sulfur atoms. The thiol group of cysteine can form a disulfide bond or a bridge with a second thiol group. In most naturally occurring IgG molecules, the CH1 and CL regions are linked by a disulfide bond.

"Chimeric antibody" refers to any antibody in which the variable region of the antibody is obtained or derived from a first species, and the constant region thereof (which may be intact, partial or modified) is derived from a second species. In certain embodiments, the variable region is derived from a non-human source (e.g., mouse or primate) and the constant region is derived from a human source.

"Specifically bind to" generally means that an antibody or an antigen-binding fragment forms a relatively stable complex with a specific antigen, through complementary binding of its antigen-binding domain and epitope. "Specificity" can be expressed by relative affinity of an antibody or an antigen-binding fragment for binding to a specific antigen or epitope. For example, an antibody "A" can be considered to have a higher specificity for an antigen than an antibody "B" if the antibody "A" has a greater relative affinity for the antigen than the antibody "B". Specific binding can be described by the equilibrium dissociation constant (KD). A smaller KD means a tighter binding. Methods for determining whether two molecules specifically bind to each other are well known in the art, and include, for example, equilibrium dialysis, surface plasmon resonance, biofilm layer interferometry, and the like. Antibodies that "specifically bind" to antigen a include antibodies that have an equilibrium dissociation constant KD of less than or equal to about 100 nM, less than or equal to about 10 nM, or less than or equal to about 5 nM with the antigen a.

"Treatment" refers to both therapeutic treatment and prophylactic or preventative measures, the purpose of which is to prevent, slow down, ameliorate, or halt undesirable physiological changes or disorders, such as the progression of a disease, including, but not limited to, alleviation of symptoms, diminishment of extent of disease, stabilized (i.e., not worsening) state of disease, delay or slowing of disease progression, amelioration, palliation, alleviation, or elimination (whether partial or total) of state of disease, prolongation of life expectancy as compared with that in the absence of treatment, and the like, as either detectable or undetectable. Patients in need of treatment include patients already with a condition or disorder, patients susceptible to a condition or disorder, or patients in need of prevention of the condition or disorder, or patients who may or are expected to benefit from administration of the antibody or the pharmaceutical composition disclosed herein for detection, diagnostic procedures, and/or treatment.

The term "cancer" means or is intended to describe the physiological state of a mammal, which is typically characterized by uncontrolled cell growth. Examples of cancers include, but are not limited to, carcinoma, lymphoma, blastoma, sarcoma, leukemia or lymphoid malignancy. More specific examples of such cancers include glioblastoma, acute myeloid leukemia (AML), non-Hodgkin's lymphoma (NHL), non-small cell lung cancer, lung cancer, colon cancer, colorectal cancer, head and neck cancer, breast cancer (e.g., triple-negative breast cancer), pancreatic cancer, squamous cell tumor, squamous cell carcinoma (e.g., squamous cell lung cancer or head and neck squamous cell carcinoma), anal cancer, skin cancer, and vulval cancer.

The term "overexpression" or "overexpressed" interchangeably refers to a gene that is transcribed or translated at a detectably greater level, usually in certain cells, e.g., a cancer cell, in comparison to a normal cell. Overexpression may be overexpression of a protein and RNA (due to increased transcription, post transcriptional processing, translation, post translational processing, altered stability, and altered protein degradation), as well as local overexpression due to altered protein traffic patterns (increased nuclear localization), and augmented functional activity, e.g., increased enzyme hydrolysis of substrate. Overexpression can be by 50%, 60%, 70%, 80%, 90% or more in comparison to a normal cell or comparison cell. In certain embodiments, the anti-B7-H3 antibodies of the present invention are used to treat solid tumors likely to overexpress B7-H3.

As used herein, the term "administering" means delivering a substance (e.g., an anti-B7-H3 antibody) for therapeutic purposes (e.g., to treat a B7-H3-related disorder). The mode of administration can be parenteral, enteral, and topical. Parenteral administration is typically performed by injection and includes, but is not limited to, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal and intrasternal injection and infusion.

As used herein, the term "effective amount" or "therapeutically effective amount" refers to the amount of a drug, e.g., an antibody, that is sufficient to reduce or ameliorate the severity and/or duration of a disorder (e.g., cancer) or one or more symptoms thereof, prevent the progression of a disorder, cause regression of a disorder, prevent the recurrence, development, onset or progression of one or more symptoms associated with a disorder, detect a disorder, or enhance or improve the prophylactic or therapeutic effect of another therapy (e.g., a prophylactic or therapeutic agent). For example, the effective amount of an antibody may inhibit tumor growth (e.g., inhibit an increase in tumor volume), decrease tumor growth (e.g., decrease tumor volume), reduce the number of cancer cells, and/or relieve to some extent one or more of the symptoms associated with the cancer. For example, the effective amount may improve disease free survival (DFS), improve overall survival (OS), or decrease likelihood of recurrence.

The terms "patient" and "subject" are used interchangeably and refer to any mammal in need of diagnosis, prognosis or treatment, including, but not limited to, humans, dogs, cats, guinea pigs, rabbits, rats, mice, horses, cattle, and the like. In some embodiments, the patient is a human being. As used herein, the term "in need" means that the patient has been identified as in need of a particular method or treatment. In some embodiments, identification may be made by any diagnostic mode. The patient may be in need of any methods and treatments described herein. The term "administration" as used herein refers to administering a substance for therapeutic purposes (e.g., treating a tumor).

The term "agent" as used herein means a chemical compound, a mixture of chemical compounds, a biological macromolecule, or an extract made from biological material.

The term "agent" or "drug" refers to a compound or composition that is capable of inducing the desired therapeutic effect when properly administered to a patient.

"About" refers to a general error range for corresponding values as readily understood by those skilled in the relevant art. In some embodiments, "about" mentioned herein refers to the values described and ranges thereof of ±10%, ±5% or ±1%.

"EC₅₀", i.e., concentration for 50% of maximal effect, refers to a concentration that causes 50% of maximal effect.

As used herein, the term "label" or "labeled" refers to incorporation of a detectable marker, e.g., by incorporation of a radiolabeled amino acid or attachment to a polypeptide of biotinyl moieties that can be detected by marked avidin (e.g., streptavidin containing a fluorescent marker or having enzymatic activity that can be detected by optical or calorimetric methods). In certain instances, the label or labels may also be therapeutic. Various methods for labeling polypeptides and glycoproteins are known in the art and can be used. Examples of labels for polypeptides include, but are not limited to: radioisotopes or radionuclides (e.g., ³H, ¹⁴C, ¹⁵N, ³⁵S, ⁹⁰Y, ⁹⁹Tc, ¹¹¹In, ¹²⁵I and ¹³¹I), fluorescent labels (e.g., FITC, rhodamine and lanthanide phosphor), enzymatic labels (e.g., horseradish peroxidase, β-galactosidase, luciferase and alkaline phosphatase), chemiluminescent labels, biotinyl groups and predetermined polypeptide epitopes recognized by secondary reporter genes (e.g., leucine zipper pair sequences, secondary antibody-binding sites, metal binding domains and epitope tags). In some embodiments, the labels are connected by spacer arms of various lengths to reduce potential steric hindrance.

The term "antibody-drug conjugate" or "ADC" refers to a binding protein (e.g., an antibody or antigen-binding fragment) that is linked to one or more chemical drugs, which may optionally be a therapeutic agent or a cytotoxic agent. In a preferred embodiment, the ADC comprises an antibody, a drug (e.g. a cytotoxic drug), and a linker capable of attaching or coupling the drug to the antibody. The number of small molecule drug binding on an antibody in ADC, i.e. the number of drug binding on an antibody, is called drug to antibody ratio (DAR). In some embodiments, the value is selected from 1, 2, 3, 4, 5, 6, 7, 8, 9, or 10. In considering the mean binding number of small molecule drugs, i.e., the mean drug binding number of an antibody, or the mean drug to antibody ratio, the value is selected from about 0 to about 10, or about 2 to about 8. In some embodiments, the drug to antibody ratio is about 3 to about 6. In other embodiments, the drug to antibody ratio is about 6 to about 8, or about 7 to about 8. Non-limiting examples of drugs that can be included in the ADC are a mitotic inhibitor, an anti-tumor antibiotic, an immunomodulator, a vector for gene therapy, an alkylating agent, an anti-angiogenic agent, an anti-metabolite, a boron-containing agent, a chemoprotective agent, a hormone, an anti-hormonal agent, a corticosteroid, a photoactive therapeutic agent, an oligonucleotide, a radionuclide agent, a topoisomerase inhibitor, a kinase inhibitor (e.g., a TEC-family kinase inhibitor and a serine/threonine kinase inhibitor) and a radiosensitizer.

The terms "antibody-drug conjugate" and "ADC" are used interchangeably. The terms "anti-B7-H3 antibody-drug conjugate" and "anti-B7-H3 ADC" are used interchangeably to refer to an ADC comprising an antibody that specifically binds to B7-H3, wherein the antibody is conjugated to one or more chemical drugs. In a preferred embodiments, the anti-B7-H3 ADC binds to human B7-H3 (hB7-H3).

Other chemical terms herein are used according to conventional usage in the art.

All the publications, patents, and patent applications cited herein are incorporated herein by reference in their entirety for all purposes.

### Anti-B7-H3 antibody

The present invention provides anti-B7-H3 antibodies or antigen-binding fragment. In some embodiments, the present invention provides an anti-B7-H3 murine antibody or an antigen-binding fragment. In some embodiments, the present invention provides an anti-B7-H3 chimeric antibody or an antigen-binding fragment. In some embodiments, the present invention provides a humanized anti-B7-H3 antibody or an antigen-binding fragment.

The anti-B7-H3 antibody or the antigen-binding fragment of the present invention has, but not limited to, in vitro binding to B7-H3, binding to cells expressing B7-H3, and strong internalization capability.

In some embodiments, the antigen-binding fragment of the anti-B7-H3 antibody is Fab, Fab', F(ab')₂, Fv, disulfide bond-linked Fv, scFv, single domain antibody, or diabody. In some embodiments, the anti-B7-H3 antibody is a multispecific antibody (e.g., bispecific antibody).

In some embodiments, the antigen-binding fragment such as Fab, F(ab')₂ and Fv can be prepared by cleavage of an intact protein, e.g., by protease or chemical cleavage, including but not limited to: (i) digesting the antibody molecule with pepsin to obtain a F(ab')₂ fragment; (ii) obtaining a Fab fragment by reducing the disulfide bond of the F(ab')₂ fragment; (iii) treating the antibody molecule with papain and a reducing agent to produce a Fab fragment, and (iv) a Fv fragment.

In some embodiments, the antibody comprises a heavy chain constant region, such as an IgG1, IgG2, IgG3, IgG4, IgA, IgE, IgM or IgD constant region. In some embodiments, the anti-B7-H3 antibody or the antigen-binding fragment comprises an immunoglobulin heavy chain constant domain selected from a human IgG constant domain, a human IgA constant domain, a human IgE constant domain, a human IgM constant domain, and a human IgD constant domain. In some embodiments, the anti-B7-H3 antibody or the antigen-binding fragment comprises an IgG1 heavy chain constant region, an IgG2 heavy chain constant region, an IgG3 heavy chain constant region, or an IgG4 heavy chain constant region. In some embodiments, the heavy chain constant region is an IgG1 heavy chain constant region or an IgG4 heavy chain constant region. In some embodiments, the antibody or the antigen-binding fragment comprises a light chain constant region, e.g., a κ light chain constant region or a λ light chain constant region. In some embodiments, the antibody or the antigen-binding fragment comprises a κ light chain constant region.

The Fc portion of an antibody mediates several important effector functions (e.g., cytokine induction, antibody-dependent cell-mediated cytotoxicity (ADCC), phagocytosis, complement-dependent cytotoxicity (CDC), and half-life/clearance of an antibody and an antigen-antibody complex). In some cases, these effector functions are desirable for therapeutic antibodies, but in other cases may be unnecessary or even detrimental depending on the therapeutic targets. Certain human IgG isotypes, particularly IgG1 and IgG3, mediate ADCC and CDC via binding to FcyRs and complement C1q, respectively. Neonatal Fc receptors (FcRns) are the critical components determining the circulating half-life of antibodies. In other embodiments, at least one amino acid residue is replaced in the constant region of the antibody (e.g., the Fc region of the antibody), such that effector functions of the antibody are altered. Replacements of amino acid residues in the Fc region of an antibody to alter antibody effector functions are described in U.S. Patent Nos. 5,648,260 and 5,624,821, which are incorporated herein by reference.

In some embodiments, the present invention includes a labeled anti-B7-H3 antibody or antigen-binding fragment, wherein the antibody is derivatized or linked to one or more functional molecules (e.g., another peptide or protein). For example, a labeled antibody can be derived by functionally linking an antibody or antigen-binding fragment of the present invention (by chemical coupling, genetic fusion, noncovalent binding or otherwise) to one or more other molecular entities, such as an addition antibody (e.g., a bispecific antibody or a bifunctional antibody), a detectable agent, a pharmaceutical agent, a protein or peptide that can mediate the binding of the antibody or antigen-binding fragment to another molecule (such as a streptavidin core region or a polyhistidine tag), and/or a cytotoxic or therapeutic agent selected from the group consisting of a mitotic inhibitor, an anti-tumor antibiotic, an immunomodulator, a vector for gene therapy, an alkylating agent, an anti-angiogenic agent, an anti-metabolite, a boron-containing agent, a chemoprotective agent, a hormone, an anti-hormonal agent, a corticosteroid, a photoactive therapeutic agent, an oligonucleotide, a radionuclide agent, a topoisomerase inhibitor, a kinase inhibitor, a radiosensitizer, and combinations thereof.

In some embodiments, the antibody or antigen-binding fragment of the present invention is linked to a detectable agent, e.g., by incorporation of a radiolabeled amino acid or attachment to a polypeptide of biotinyl moieties that can be detected by marked avidin (e.g., streptavidin containing a fluorescent marker or having enzymatic activity that can be detected by optical or calorimetric methods). In certain instances, the label or labels may also be therapeutic. Various methods for labeling polypeptides and glycoproteins are known in the art and can be used. Examples of labels for polypeptides include, but are not limited to: radioisotopes or radionuclides (e.g., ³H, ¹⁴C, ¹⁵N, ³⁵S, ⁹⁰Y, ⁹⁹Tc, ¹¹¹In, ¹²⁵I and ¹³¹I), fluorescent labels (e.g., FITC, rhodamine and lanthanide phosphor), enzymatic labels (e.g., horseradish peroxidase, β-galactosidase, luciferase and alkaline phosphatase), chemiluminescent labels, biotinyl groups and predetermined polypeptide epitopes recognized by secondary reporter genes (e.g., leucine zipper pair sequences, secondary antibody-binding sites, metal binding domains and epitope tags). In some embodiments, the labels are connected by spacer arms of various lengths to reduce potential steric hindrance.

In other embodiments, the antibody or the antigen-binding fragment of the present invention can be used to detect whether the B7-H3 (e.g., human B7-H3) or a fragment thereof is present in a sample. In some embodiments, the antibody comprises a detectable agent. The antibody is a polyclonal antibody, or more preferably, a monoclonal antibody. An intact antibody or antigen-binding fragment (e.g., Fab, scFv, or F(ab')₂) can be used. The detection method can be used to detect an analyte mRNA, protein or genomic DNA in a biological sample *in vitro* as well as *in vivo.* For example, *in-vitro* techniques for detection of an analyte mRNA include Northern hybridization and in situ hybridization. *In-vitro* techniques for detection of an analyte protein include enzyme linked immunosorbent assay (ELISA), Western blots, immunoprecipitation, and immunofluorescence. *In-vitro* techniques for detection of an analyte genomic DNA include Southern hybridization. In addition, *in-vivo* techniques for detection of an analyte protein include introducing a labeled anti-analyte protein antibody into a patient. For example, the antibody can be labeled with a radioactive marker, the presence and location of which in the patient can then be detected by standard imaging techniques.

The anti-B7-H3 antibody disclosed herein can be monoclonal antibodies.

The binding specificity of the antibody or the antigen-binding fragment disclosed herein can be detected by *in-vitro* assays, such as co-immunoprecipitation, radioimmunoassay (RIA), surface plasmon resonance, flow cytometry (Facs), or enzyme-linked immunosorbent assay (ELISA). The present invention further comprises an antibody that binds to the same epitope as the anti-B7-H3 antibody described herein. For example, the antibody of the present invention specifically binds to an epitope comprising one or more amino acid residues on human B7-H3.

An exemplary amino acid sequence SEQ ID NO: 44 (GenBank accession No. NP_001019907.1, which is incorporated herein by reference) of human B7-H3 is provided below. The signal sequence (amino acids 1-28) is underlined and italicized.
SEQ ID NO: 44:

MLRRRGSPGMGVHVGAALGALWFCLTGALEVQVPEDPVVALVGTDATLCCSFSPEPGFSL
AQLNLIWQLTDTKQLVHSFAEGQDQGSAYANRTALFPDLLAQGNASLRLQRVRVADEGS
FTCFVSIRDFGSAAVSLQVAAPYSKPSMTLEPNKDLRPGDTVTITCSSYQGYPEAEVFWQ
DGQGVPLTGNVTTSQMANEQGLFDVHSILRVVLGANGTYSCLVRNPVLQQDAHSSVTIT
PQRSPTGAVEVQVPEDPVVALVGTDATLRCSFSPEPGFSLAQLNLIWQLTDTKQLVHSFTE
GRDQGSAYANRTALFPDLLAQGNASLRLQRVRVADEGSFTCFVSIRDFGSAAVSLQVAAP
YSKPSMTLEPNKDLRPGDTVTITCSSYRGYPEAEVFWQDGQGVPLTGNVTTSQMANEQ
GLFDVHSVLRVVLGANGTYSCLVRNPVLQQDAHGSVTITGQPMTFPPEALWVTVGLSV
CLIALLVALAFVCWRKIKQSCEEENAGAEDQDGEGEGSKTALQPLKHSDSKEDDGQEIA

### 1) Anti-B7-H3 murine antibody

The heavy chain variable region of an exemplary anti-B7-H3 murine antibody of the present invention comprises an amino acid sequence set forth in SEQ ID NO: 24 or an amino acid sequence having suitable sequence identity, such as at least 80% sequence identity, or at least 90% sequence identity, or at least 95% sequence identity, or at least 99% sequence identity, compared with the amino acid sequence set forth in SEQ ID NO: 24; the light chain variable region comprises an amino acid sequence set forth in any one of SEQ ID NOs: 25-29 or an amino acid sequence having suitable sequence identity, such as at least 80% sequence identity, or at least 90% sequence identity, or at least 95% sequence identity, or at least 99% sequence identity, compared with the amino acid sequence set forth in any one of SEQ ID NOs: 25-29. In some embodiments, these amino acid sequences having suitable sequence identity are at least CDR invariant.

### 2) Anti-B7-H3 chimeric antibody

In some embodiments, the present invention provides an anti-B7-H3 chimeric antibody in which the variable regions of an anti-B7-H3 murine antibody is linked to a human immunoglobulin constant region.

In some embodiments, the anti-B7-H3 chimeric antibody or the antigen-binding fragment of the present invention comprises a heavy chain variable region comprising an amino acid sequence set forth in SEQ ID NO: 24 or an amino acid sequence having suitable sequence identity, such as at least 80% sequence identity, or at least 90% sequence identity, or at least 95% sequence identity, or at least 99% sequence identity, compared with the amino acid sequence set forth in SEQ ID NO: 24. In some embodiments, these amino acid sequences having sequence identity are at least CDR invariant.

In some embodiments, the anti-B7-H3 chimeric antibody or the antigen-binding fragment of the present invention comprises a light chain variable region comprising an amino acid sequence set forth in any one of SEQ ID NOs: 25-29 or an amino acid sequence having suitable sequence identity, such as at least 80% sequence identity, or at least 90% sequence identity, or at least 95% sequence identity, or at least 99% sequence identity, compared with the amino acid sequence set forth in any one of SEQ ID NOs: 25-29. In some embodiments, these amino acid sequences having sequence identity are at least CDR invariant.

In some embodiments, the anti-B7-H3 chimeric antibody or the antigen-binding fragment of the present invention comprises a heavy chain variable region having an amino acid sequence set forth in SEQ ID NO: 24 and a light chain variable region having an amino acid sequence set forth in SEQ ID NO: 25.

In some embodiments, the anti-B7-H3 chimeric antibody or the antigen-binding fragment of the present invention comprises: a heavy chain variable region comprising (a) a VH CDR1 having an amino acid sequence set forth in SEQ ID NO: 6, (b) a VH CDR2 having an amino acid sequence set forth in SEQ ID NO: 7, and (c) a VH CDR3 having an amino acid sequence set forth in SEQ ID NO: 8; and a light chain variable region comprising (a) a VL CDR1 having an amino acid sequence set forth in SEQ ID NO: 9, (b) a VL CDR2 having an amino acid sequence set forth in SEQ ID NO: 14, and (c) a VL CDR3 having an amino acid sequence set forth in SEQ ID NO: 19. In some embodiments, the anti-B7-H3 chimeric antibody or the antigen-binding fragment of the present invention comprises a heavy chain variable region having an amino acid sequence set forth in SEQ ID NO: 24 and a light chain variable region having an amino acid sequence set forth in SEQ ID NO: 26.

In some embodiments, the anti-B7-H3 chimeric antibody or the antigen-binding fragment of the present invention comprises: a heavy chain variable region comprising (a) a VH CDR1 having an amino acid sequence set forth in SEQ ID NO: 6, (b) a VH CDR2 having an amino acid sequence set forth in SEQ ID NO: 7, and (c) a VH CDR3 having an amino acid sequence set forth in SEQ ID NO: 8; and a light chain variable region comprising (a) a VL CDR1 having an amino acid sequence set forth in SEQ ID NO: 10, (b) a VL CDR2 having an amino acid sequence set forth in SEQ ID NO: 15, and (c) a VL CDR3 having an amino acid sequence set forth in SEQ ID NO: 20. In some embodiments, the anti-B7-H3 chimeric antibody or the antigen-binding fragment of the present invention comprises a heavy chain variable region having an amino acid sequence set forth in SEQ ID NO: 24 and a light chain variable region having an amino acid sequence set forth in SEQ ID NO: 27.

In some embodiments, the anti-B7-H3 chimeric antibody or the antigen-binding fragment of the present invention comprises: a heavy chain variable region comprising (a) a VH CDR1 having an amino acid sequence set forth in SEQ ID NO: 6, (b) a VH CDR2 having an amino acid sequence set forth in SEQ ID NO: 7, and (c) a VH CDR3 having an amino acid sequence set forth in SEQ ID NO: 8; and a light chain variable region comprising (a) a VL CDR1 having an amino acid sequence set forth in SEQ ID NO: 11, (b) a VL CDR2 having an amino acid sequence set forth in SEQ ID NO: 16, and (c) a VL CDR3 having an amino acid sequence set forth in SEQ ID NO: 21. In some embodiments, the anti-B7-H3 chimeric antibody or the antigen-binding fragment of the present invention comprises a heavy chain variable region having an amino acid sequence set forth in SEQ ID NO: 24 and a light chain variable region having an amino acid sequence set forth in SEQ ID NO: 28.

In some embodiments, the anti-B7-H3 chimeric antibody or the antigen-binding fragment of the present invention comprises: a heavy chain variable region comprising (a) a VH CDR1 having an amino acid sequence set forth in SEQ ID NO: 6, (b) a VH CDR2 having an amino acid sequence set forth in SEQ ID NO: 7, and (c) a VH CDR3 having an amino acid sequence set forth in SEQ ID NO: 8; and a light chain variable region comprising (a) a VL CDR1 having an amino acid sequence set forth in SEQ ID NO: 12, (b) a VL CDR2 having an amino acid sequence set forth in SEQ ID NO: 17, and (c) a VL CDR3 having an amino acid sequence set forth in SEQ ID NO: 22. In some embodiments, the anti-B7-H3 chimeric antibody or the antigen-binding fragment of the present invention comprises a heavy chain variable region having an amino acid sequence set forth in SEQ ID NO: 24 and a light chain variable region having an amino acid sequence set forth in SEQ ID NO: 29.

In some embodiments, the anti-B7-H3 chimeric antibody or the antigen-binding fragment of the present invention comprises: a heavy chain variable region comprising (a) a VH CDR1 having an amino acid sequence set forth in SEQ ID NO: 6, (b) a VH CDR2 having an amino acid sequence set forth in SEQ ID NO: 7, and (c) a VH CDR3 having an amino acid sequence set forth in SEQ ID NO: 8; and a light chain variable region comprising (a) a VL CDR1 having an amino acid sequence set forth in SEQ ID NO: 13, (b) a VL CDR2 having an amino acid sequence set forth in SEQ ID NO: 18, and (c) a VL CDR3 having an amino acid sequence set forth in SEQ ID NO: 23. In some embodiments, the anti-B7-H3 chimeric antibody or the antigen-binding fragment of the present invention further comprises a heavy chain constant region comprising an amino acid sequence set forth in SEQ ID NO: 32 or 33 or an amino acid sequence having suitable sequence identity, such as at least 80% sequence identity, or at least 90% sequence identity, or at least 95% sequence identity, or at least 99% sequence identity, compared with the amino acid sequence set forth in SEQ ID NO: 32 or 33.

In some embodiments, the anti-B7-H3 chimeric antibody or the antigen-binding fragment of the present invention further comprises a light chain constant region comprising an amino acid sequence set forth in SEQ ID NO: 34 or an amino acid sequence having suitable sequence identity, such as at least 80% sequence identity, or at least 90% sequence identity, or at least 95% sequence identity, or at least 99% sequence identity, compared with the amino acid sequence set forth in SEQ ID NO: 34.

In some embodiments, the heavy chain of the anti-B7-H3 chimeric antibody or the antigen-binding fragment of the present invention further comprises a signal peptide, e.g., MEFGLSWVFLVAILKGVQC (SEQ ID NO: 45) or MKHLWFFLLLVAAPRWVLS (SEQ ID NO: 46). In some embodiments, the light chain of the anti-B7-H3 chimeric antibody or the antigen-binding fragment of the present invention further comprises a signal peptide, e.g., MDMRVLAQLLGLLLLCFPGARC (SEQ ID NO: 47) or MVLQTQVFISLLLWISGAYG (SEQ ID NO: 48).

In some embodiments, the anti-B7-H3 chimeric antibody or the antigen-binding fragment of the present invention binds to B7-H3 with an equilibrium dissociation constant (KD) of about 1 µM or less. In some embodiments, the anti-B7-H3 chimeric antibody or the antigen-binding fragment of the present invention binds to B7-H3 with a KD of between about 1 µM and about 1 pM. In some embodiments, the anti-B7-H3 chimeric antibody or the antigen-binding fragment of the present invention binds to B7-H3 with a KD of between about 100 nM and about 1 pM. In some embodiments, the anti-B7-H3 chimeric antibody or the antigen-binding fragment of the present invention binds to B7-H3 with a KD of between about 10 nM and about 1 pM. In some embodiments, the anti-B7-H3 chimeric antibody or the antigen-binding fragment of the present invention binds to B7-H3 with a KD of between about 1 nM and about 0.1 nM.

In some embodiments, the anti-B7-H3 chimeric antibody or the antigen-binding fragment of the present invention binds to hB7-H3-His (set forth in SEQ ID NO: 4) with a KD of about 1 µM or less. In some embodiments, the anti-B7-H3 chimeric antibody or the antigen-binding fragment of the present invention binds to hB7-H3-His (set forth in SEQ ID NO: 4) with a KD of between about 1 µM and about 1 pM, or between about 100 nM and about 1 pM, or between about 10 nM and about 1 pM, or between about 1 nM and about 1 pM, or between about 1 nM and about 0.1 nM, or between about 0.5 nM and about 0.3 nM.

The murine antibody or the chimeric antibody disclosed herein can be used to generate a humanized anti-B7-H3 antibody. For example, the chimeric antibody M1 is selected for humanization.

### 3) Humanized anti-B7-H3 antibody

The antibodies described herein include humanized antibodies. These antibodies are suitable for administration to an animal (e.g., a human being) without eliciting an adverse immune response in the animal (e.g., a human being) to be treated.

In some embodiments, the humanized anti-B7-H3 antibody or the antigen-binding fragment of the present invention comprises: a heavy chain variable region comprising (a) a VH CDR1 having an amino acid sequence set forth in SEQ ID NO: 6, (b) a VH CDR2 having an amino acid sequence set forth in SEQ ID NO: 7, and (c) a VH CDR3 having an amino acid sequence set forth in SEQ ID NO: 8; and a light chain variable region comprising (a) a VL CDR1 having an amino acid sequence set forth in SEQ ID NO: 9, (b) a VL CDR2 having an amino acid sequence set forth in SEQ ID NO: 14, and (c) a VL CDR3 having an amino acid sequence set forth in SEQ ID NO: 19. In some embodiments, the heavy chain variable region of the humanized anti-B7-H3 antibody or the antigen-binding fragment of the present invention comprises an amino acid sequence set forth in any one of SEQ ID NOs: 35-38 or an amino acid sequence having suitable sequence identity, such as at least 80% sequence identity, or at least 90% sequence identity, or at least 95% sequence identity, or at least 99% sequence identity, compared with the amino acid sequence set forth in any one of SEQ ID NOs: 35-38; and/or its light chain variable region comprises an amino acid sequence set forth in any one of SEQ ID NOs: 39-41 or an amino acid sequence having suitable sequence identity, such as at least 80% sequence identity, or at least 90% sequence identity, or at least 95% sequence identity, or at least 99% sequence identity, compared with the amino acid sequence set forth in any one of SEQ ID NOs: 39-41. In some embodiments, these amino acid sequences having sequence identity are at least CDR invariant.

In some embodiments, the humanized anti-B7-H3 antibody or the antigen-binding fragment of the present invention comprises a heavy chain variable region having an amino acid sequence set forth in SEQ ID NO: 35 and a light chain variable region having an amino acid sequence set forth in SEQ ID NO: 39.

In some embodiments, the humanized anti-B7-H3 antibody or the antigen-binding fragment of the present invention comprises a heavy chain variable region having an amino acid sequence set forth in SEQ ID NO: 36 and a light chain variable region having an amino acid sequence set forth in SEQ ID NO: 39.

In some embodiments, the humanized anti-B7-H3 antibody or the antigen-binding fragment of the present invention comprises a heavy chain variable region having an amino acid sequence set forth in SEQ ID NO: 36 and a light chain variable region having an amino acid sequence set forth in SEQ ID NO: 40.

In some embodiments, the humanized anti-B7-H3 antibody or the antigen-binding fragment of the present invention comprises a heavy chain variable region having an amino acid sequence set forth in SEQ ID NO: 37 and a light chain variable region having an amino acid sequence set forth in SEQ ID NO: 41.

In some embodiments, the humanized anti-B7-H3 antibody or the antigen-binding fragment of the present invention comprises a heavy chain variable region having an amino acid sequence set forth in SEQ ID NO: 38 and a light chain variable region having an amino acid sequence set forth in SEQ ID NO: 41.

In some embodiments, the humanized anti-B7-H3 antibody or the antigen-binding fragment of the present invention further comprises a heavy chain constant region comprising an amino acid sequence set forth in SEQ ID NO: 32 or 33 or an amino acid sequence having suitable sequence identity, such as at least 80% sequence identity, or at least 90% sequence identity, or at least 95% sequence identity, or at least 99% sequence identity, compared with the amino acid sequence set forth in SEQ ID NO: 32 or 33.

In some embodiments, the humanized anti-B7-H3 antibody or the antigen-binding fragment of the present invention further comprises a light chain constant region comprising an amino acid sequence set forth in SEQ ID NO: 34 or an amino acid sequence having suitable sequence identity, such as at least 80% sequence identity, or at least 90% sequence identity, or at least 95% sequence identity, or at least 99% sequence identity, compared with the amino acid sequence set forth in SEQ ID NO: 34.

In some embodiments, the heavy chain of the humanized anti-B7-H3 antibody or the antigen-binding fragment of the present invention further comprises a signal peptide, e.g., MEFGLSWVFLVAILKGVQC (SEQ ID NO: 45) or MKHLWFFLLLVAAPRWVLS (SEQ ID NO: 46). In some embodiments, the light chain of the humanized anti-B7-H3 antibody or the antigen-binding fragment of the present invention further comprises a signal peptide, e.g., MDMRVLAQLLGLLLLCFPGARC (SEQ ID NO: 47) or MVLQTQVFISLLLWISGAYG (SEQ ID NO: 48).

In some embodiments, the humanized anti-B7-H3 antibody or the antigen-binding fragment of the present invention binds to B7-H3 with an equilibrium dissociation constant (KD) of about 1 µM or less. In some embodiments, the humanized anti-B7-H3 antibody or the antigen-binding fragment of the present invention binds to B7-H3 with a KD of between about 1 µM and about 1 pM. In some embodiments, the humanized anti-B7-H3 antibody or the antigen-binding fragment of the present invention binds to B7-H3 with a KD of between about 100 nM and about 1 pM. In some embodiments, the humanized anti-B7-H3 antibody or the antigen-binding fragment of the present invention binds to B7-H3 with a KD of between about 10 nM and about 1 pM. In some embodiments, the humanized anti-B7-H3 antibody or the antigen-binding fragment of the present invention binds to B7-H3 with a KD of between about 1 nM and about 0.1 nM.

In some embodiments, the humanized anti-B7-H3 antibody or the antigen-binding fragment of the present invention binds to hB7-H3-His (set forth in SEQ ID NO: 4) with a KD of about 1 µM or less. In some embodiments, the humanized anti-B7-H3 antibody or the antigen-binding fragment of the present invention binds to hB7-H3-His (set forth in SEQ ID NO: 4) with a KD of between about 1 µM and about 1 pM, or between about 100 nM and about 1 pM, or between about 10 nM and about 1 pM, or between about 1 nM and about 1 pM, or between about 1 nM and about 0.1 nM, or between about 0.5 nM and about 0.3 nM.

### Treatment method

The antibody or the antigen-binding fragment of the present invention is capable of neutralizing B7-H3 activity *in vivo* and *in vitro.* Thus, the antibody or the antigen-binding fragment of the present invention can be used to inhibit B7-H3 activity, for example in a cell culture containing B7-H3, in human patients or in other mammals having B7-H3 with which the antibody of the present invention cross-reacts. In one embodiment, the present invention provides a method for inhibiting B7-H3 activity, which comprises contacting B7-H3 with the antibody of the present invention, thereby inhibiting B7-H3 activity. For example, in a cell culture containing or suspected of containing B7-H3, the antibody or the antigen-binding fragment of the present invention can be added to the medium to inhibit B7-H3 activity in the culture. In some embodiments, the B7-H3 is human B7-H3, and the patient is a human patient.

In some embodiments, provided is a method for reducing B7-H3 activity in a patient who suffers from a disease or disorder in which B7-H3 activity is detrimental. The present invention provides a method for reducing B7-H3 activity in a patient suffering from such a disease or disorder, and the method comprises administering to the patient an effective dose of the antibody or the antigen-binding fragment of the present invention, such that B7-H3 activity in the patient is reduced. In some embodiments, the B7-H3 is human B7-H3, and the patient is a human patient. Alternatively, the patient may be a mammal expressing B7-H3 to which the antibody of the present invention is capable of binding. In addition, the patient may be a mammal into which B7-H3 has been introduced (e.g., by administration of B7-H3 or by expression of a B7-H3 transgene). The antibody or the antigen-binding fragment of the present invention may be administered to a human patient for therapeutic purposes. Furthermore, the antibody or the antigen-binding fragment of the present invention may be administered to a non-human mammal expressing B7-H3 to which the antibody is capable of binding (for veterinary purposes or as an animal model of a human disease). Such animal models of human disease can be used to assess the therapeutic efficacy (e.g., dose testing and time course of administration) of the antibody or the antigen-binding fragment of the present invention.

In some embodiments, provided is a method for preventing, treating or ameliorating various types of tumors (including benign tumors and cancers) and related diseases, which comprises administering to a patient an effective amount of the antibody or the antigen-binding fragment. In some embodiments, provided is use of the antibody or the antigen-binding fragment in preventing, treating or ameliorating tumors (including benign tumors and cancers) and related diseases. In some embodiments, provided is use of the antibody or the antigen-binding fragment in the manufacture of a drug for preventing, treating or ameliorating tumors (including benign tumors and cancers) and related diseases. In some embodiments, the tumor (including benign tumors and cancers) is a tumor expressing B7-H3 (including benign tumors and cancers). In some embodiments, the tumor (including benign tumors and cancers) is a tumor overexpressing B7-H3 (including benign tumors and cancers). Methods for identifying a tumor expressing B7-H3 (e.g., a tumor overexpressing B7-H3) are known in the art. For example, expression of B7-H3 in normal and tumor tissues is detected by immunohistochemistry.

In some embodiments, the present invention relates to: a method for treating related diseases with B7-H3 as a therapeutic target, thereby ameliorating, slowing, inhibiting, or preventing any disease or disorder associated with overexpression of B7-H3; a method for treating tumors (including benign tumors and cancers) in a patient, a method for alleviating symptoms of tumors (including benign tumors and cancers) in a patient, and a method for avoiding recurrence of tumors (including benign tumors and cancers) in a patient, which comprise administering to the patient an effective amount of any of the antibodies or the antigen-binding fragments described herein.

The antibody or the antigen-binding fragment and the pharmaceutical composition comprising the same provided herein can be used as a therapeutic agent for diagnosis, prognosis, monitoring, treatment, alleviation and/or prevention of diseases and disorders related to abnormal expression, activity and/or signaling of B7-H3 in a patient. When a disease and a disorder related to abnormal expression, activity and/or signaling of B7-H3 in a patient are identified by using a standard method, the antibody or the antigen-binding fragment and the pharmaceutical composition comprising the same disclosed herein may be administered.

In some embodiments, cancers treated and/or prevented with the antibody or the antigen-binding fragment described herein include, but are not limited to, solid tumors, hematological tumors, and metastatic lesions. Examples of cancers include, but are not limited to, carcinoma, blastoma, sarcoma, or leukemia. More specific examples of such cancers include, but are not limited to, melanoma, lung cancer (e.g., small cell lung cancer and non-small cell lung cancer), breast cancer (e.g., triple-negative breast cancer), ovarian cancer (e.g., ovarian epithelial carcinoma), glioma (e.g., glioblastoma or childhood brain stem glioma), prostate cancer (e.g., castration-resistant prostate cancer), pancreatic cancer (e.g., pancreatic ductal carcinoma), head and neck cancer, leukemia (e.g., acute myeloid leukemia (AML)), cervical cancer, kidney cancer, squamous cell tumor, squamous cell carcinoma (e.g., squamous cell lung cancer or head and neck squamous cell carcinoma), colorectal cancer, gastric cancer, liver cancer, mesothelioma, anal cancer, skin cancer, vulval cancer, neuroblastoma, desmoplastic small round cell tumor, medulloblastoma, meningioma, peritoneal malignancy, sarcoma, brain cancer, central nervous system tumor and metastatic brain tumor. In some embodiments, the antibody or the antigen-binding fragment of the present invention is administered to a patient who suffers from one or more tumors (including benign tumors or cancers) and overexpresses B7-H3. In some embodiments, the antibody or the antigen-binding fragment of the present invention is administered to a patient who suffers from a solid tumor and may overexpress B7-H3. In some embodiments, the antibody or the antigen-binding fragment of the present invention is administered to a patient suffering from squamous cell non-small cell lung cancer (NSCLC). In some embodiments, the antibody or the antigen-binding fragment of the present invention is administered to a patient having a solid tumor (including advanced solid tumors). In some embodiments, the antibody or the antigen-binding fragment of the present invention is administered to a patient suffering from prostate cancer. In some embodiments, the antibody or the antigen-binding fragment of the present invention is administered to a patient suffering from non-small cell lung cancer. In some embodiments, the antibody or the antigen-binding fragment of the present invention is administered to a patient suffering from glioblastoma. In some embodiments, the antibody or the antigen-binding fragment of the present invention is administered to a patient suffering from colon cancer. In some embodiments, the antibody or the antigen-binding fragment of the present invention is administered to a patient suffering from head and neck cancer. In some embodiments, the antibody or the antigen-binding fragment of the present invention is administered to a patient suffering from kidney cancer. In some embodiments, the antibody or the antigen-binding fragment of the present invention is administered to a patient suffering from clear cell renal cell carcinoma. In some embodiments, the antibody or the antigen-binding fragment of the present invention is administered to a patient suffering from glioma. In some embodiments, the antibody or the antigen-binding fragment of the present invention is administered to a patient suffering from melanoma. In some embodiments, the antibody or the antigen-binding fragment of the present invention is administered to a patient suffering from pancreatic cancer. In some embodiments, the antibody or the antigen-binding fragment of the present invention is administered to a patient suffering from gastric cancer. In some embodiments, the antibody or the antigen-binding fragment of the present invention is administered to a patient suffering from ovarian cancer. In some embodiments, the antibody or the antigen-binding fragment of the present invention is administered to a patient suffering from colorectal cancer. In some embodiments, the antibody or the antigen-binding fragment of the present invention is administered to a patient suffering from small cell lung cancer. In some embodiments, the antibody or the antigen-binding fragment of the present invention is administered to a patient suffering from hypopharyngeal squamous cell carcinoma. In some embodiments, the antibody or the antigen-binding fragment of the present invention is administered to a patient suffering from neuroblastoma. In some embodiments, the antibody or the antigen-binding fragment of the present invention is administered to a patient suffering from breast cancer. In some embodiments, the antibody or the antigen-binding fragment of the present invention is administered to a patient suffering from endometrial cancer. In some embodiments, the antibody or the antigen-binding fragment of the present invention is administered to a patient suffering from urothelial cell carcinoma. In some embodiments, the antibody or the antigen-binding fragment of the present invention is administered to a patient suffering from acute myeloid leukemia (AML).

The specific dose and regimen for any particular patient will depend on a variety of factors including the particular antibody or derivative thereof used, the age and body weight, general health condition, sex and diet of the patient, and the time of administration, frequency of excretion, drug combination and the severity of the particular disease being treated. These factors are judged by medical caregivers included within the scope of those of ordinary skills in the art. The dose will also depend on the individual patient to be treated, the route of administration, the type of the formulation, the nature of the compound used, the severity of the disease and the efficacy desired. The dose employed can be determined by pharmacological and pharmacokinetic principles well known in the art. In some embodiments, an effective dose ranges from about 0.01 mg/kg to about 100 mg/kg, and can be administered, for example, once a day to once a month. For example, the administration may be performed by intravenous infusion, intravenous bolus injection, subcutaneous injection, intramuscular injection, etc. It should be noted that dose values may vary with the type and severity of the condition to be alleviated. It is to be further understood that for any particular patient, specific dosage regimens should be adjusted over time according to the patient's need and the professional judgment of the person administering or supervising the administration of the compositions, and that dose ranges set forth herein are exemplary only and are not intended to limit the scope or practice of the claimed composition.

The modes of administration for the antibody or the antigen-binding fragment include, but are not limited to, intradermal, intramuscular, intraperitoneal, intravenous, subcutaneous, nasal, epidural and oral administration. The pharmaceutical composition may be administered by any convenient route, e.g., by infusion or bolus injection, by absorption through epithelial or cutaneous mucosa (e.g., oral mucosa or rectal and intestinal mucosa), and may be co-administered with other biologically active agents. Thus, the pharmaceutical composition comprising the antibody of the present invention can be administered orally, rectally, parenterally, intracisternally, intravaginally, intraperitoneally, topically (e.g. by powder, ointment, drop or transdermal patch) or buccally, or by nasal spray.

The term "parenteral" as used herein refers to modes of administration including intravenous, intramuscular, intraperitoneal, intrasternal, subcutaneous and intraarticular injections and infusions.

The mode of administration may be systemic or local. In addition, it may be desirable to introduce the antibody or the antigen-binding fragment of the present invention into the central nervous system by any suitable route, including intracerebroventricular and intrathecal injections; intracerebroventricular injection may be assisted by an intracerebroventricular catheter connected to, for example, a reservoir (which may be an Ommaya reservoir). Pulmonary administration is also possible, for example by use of an inhaler or nebulizer, and by use of nebulized formulations. The antibody or the antigen-binding fragment of the present invention may be administered locally to an area in need of treatment; this may be achieved by (but not limited to) the following: local infusion during surgery (e.g., topical application in combination with a post-operative wound dressing), by injection, by means of a catheter, by means of a suppository, or by means of an implant, the implant being of a porous, non-porous, or gelatinous material, including membranes (such as silicone rubber membranes) or fibers. Preferably, when administering the antibody or the antigen-binding fragment of the present invention, care must be taken to use materials that do not absorb protein.

Methods for treating diseases comprising administering the antibody or derivatives described herein are generally tested *in vitro,* followed by *in-vivo* tests for desired therapeutic or prophylactic activities in an acceptable animal model, and finally administration in human beings. Suitable animal models (including transgenic animals) are well known to those of ordinary skill in the art. For example, *in-vitro* assays for demonstrating therapeutic use of the antibody or the antigen-binding fragment of the present invention include the effect of the antibody on a cell line or a patient tissue sample. The effect of the antibody on the cell line and/or the tissue sample can be detected using techniques known to those skilled in the art, such as the techniques disclosed elsewhere herein. In accordance with the teachings of the present invention, *in-vitro* assays that can be used to determine whether to administer the antibody or the antigen-binding fragment include *in-vitro* cell culture experiments, wherein a patient tissue sample is cultured in a culture medium and is exposed to or otherwise administered with the antibody or the antigen-binding fragment, and the effect of the antibody or the antigen-binding fragment on the tissue sample is observed.

Various known delivery systems can be used to administer the antibody or the antigen-binding fragment, or the polynucleotide encoding the same disclosed herein, e.g., encapsulation in liposomes, microparticles, microcapsules, recombinant cells capable of expressing the compound, receptor-mediated endocytosis, construction of nucleic acids as part of a retrovirus or other vectors.

### Combination therapy

In some embodiments, the antibody or the antigen-binding fragment of the present invention can be administered in combination with other therapeutic or prophylactic regimens, including administration of one or more antibodies or antigen-binding fragments of the present invention together with or in combination with one or more other therapeutic agents or methods. For combination therapy, the antibody or the antigen-binding fragment may be administered simultaneously or separately with other therapeutic agents. When administered separately, the antibody or the antigen-binding fragment of the present invention can be administered before or after administration of another therapeutic agent.

In some embodiments, when the antibody or the antigen-binding fragment of the present invention is administered to a patient, the antibody molecules or the pharmaceutical composition disclosed herein and one or more other therapies, such as therapeutic modalities and/or other formulations (e.g., a therapeutic agent), may also be administered in combination to the patient.

Such combination therapies encompass both simultaneous administration (wherein two or more formulations are contained in the same formulation or separate formulations) and separate administration (wherein the antibody or the antigen-binding fragment of the present invention can be administered prior to, concurrently with, and/or subsequent to the administration of other therapies, e.g., therapeutic modalities or therapeutic agents). The antibody molecules and/or other therapies, e.g., therapeutic agents or therapeutic modalities, can be administered when a disease is active or when the disease is in remission or less active. The antibody molecules can be administered prior to, concurrently with or subsequent to other therapies, or during remission of a disease.

In some embodiments, the antibody or the antigen-binding fragment of the present invention is administered in combination with a therapeutic agent. Therapeutic agents include, but are not limited to: a cytokine and growth factor inhibitor, an immunosuppressant, an anti-inflammatory agent (e.g., a systemic anti-inflammatory agent), an anti-fibrotic agent, a metabolic inhibitor, an enzyme inhibitor and/or a cytotoxic or cytostatic agent, a mitotic inhibitor, an anti-tumor antibiotic, an immunomodulator, a vector for gene therapy, an alkylating agent, an anti-angiogenic agent, an anti-metabolite, a boron-containing agent, a chemoprotective agent, a hormone, an anti-hormonal agent, a corticosteroid, a photoactive therapeutic agent, an oligonucleotide, a radionuclide agent, a topoisomerase inhibitor, a kinase inhibitor or a radiosensitizer.

In some embodiments, the antibody or the antigen-binding fragment of the present invention is administered in combination with an anti-cancer agent or an anti-tumor agent. The terms "anti-cancer agent" and "anti-neoplastic agent" refer to drugs used to treat malignant tumors, e.g., to inhibit cancerous growth. For example, breast cancer is often stimulated by estrogens and can be treated with drugs that inactivate sex hormones. Similarly, prostate cancer can be treated with drugs that inactivate androgens. Examples of anti-cancer agents include, but are not limited to: an anti-PD1 antibody (e.g., pembrolizumab), an anti-PD-L1 antibody (e.g., atezolizumab), an anti-CTLA-4 antibody (e.g., ipilimumab), a MEK inhibitor (e.g., trametinib), an ERK inhibitor, a BRAF inhibitor (e.g., dabrafenib), osimertinib, erlotinib, gefitinib, sorafenib, a CDK9 inhibitor (e.g.,dinaciclib), an MCL-1 inhibitor, temozolomide, a Bcl-xL inhibitor, a Bcl-2 inhibitor (e.g., venetoclax), ibrutinib, an mTOR inhibitor (e.g., everolimus), a PI3K inhibitor (e.g., buparlisib), duvelisib, idelalisib, an AKT inhibitor, an HER2 inhibitor (e.g., lapatinib), herceptin, a taxane (e.g., docetaxel, paclitaxel or nab-paclitaxel), an ADC comprising auristatin, an ADC comprising PBD (e.g., rovalpituzumab tesirine), an ADC comprising maytansinoid (e.g., TDM1), a TRAIL agonist, a proteasome inhibitor (e.g., bortezomib), a nicotinamide phosphoribosyltransferase inhibitor, panorex, rituximab, gemtuzumab, alemtuzumab, ibritumomab, tositumomab, cetuximab, avastin, and herceptin.

Examples of cytokines that can be administered in combination with the antibody or the antigen-binding fragment of the present invention include, but are not limited to, one or more of the following: IL-1, IL-2, IL-3, IL-4, IL-5, IL-6, IL-7, IL-8, IL-15, IL-16, IL-18, IL-21, IL-31, and the like.

Examples of other therapeutic agents that may be administered in combination with the antibody or the antigen-binding fragment of the present invention include, but are not limited to, one or more of the following: an inhaled steroid; a β-agonist, e.g., a short-acting or long-acting β agonist; an antagonist of leukotriene or leukotriene receptor; a combination drug, such as ADVAIR; an IgE inhibitor, e.g., an anti-IgE antibody (e.g., omalizumab); a phosphodiesterase inhibitor (e.g., a PDE4 inhibitor); xanthine; an anticholinergic agent; a mast cell stabilizer, such as cromolyn; an IL-4 inhibitor; an IL-5 inhibitor; an eotaxin/CCR3 inhibitor; an antagonist of histamine or its receptors (including H1, H2, H3 and H4), and an antagonist of prostaglandin D or its receptors (DP1 and CRTH2); an anti-PD1 antibody (e.g., pembrolizumab), an anti-PD-L1 antibody (e.g., atezolizumab), an anti-CTLA-4 antibody (e.g., ipilimumab), a MEK inhibitor (e.g., trametinib), an ERK inhibitor, a BRAF inhibitor (e.g., dabrafenib), osimertinib, erlotinib, gefitinib, sorafenib, a CDK9 inhibitor (e.g.,dinaciclib), an MCL-1 inhibitor, temozolomide, a Bcl-xL inhibitor, a Bcl-2 inhibitor (e.g., venetoclax), ibrutinib, an mTOR inhibitor (e.g., everolimus), a PI3K inhibitor (e.g., buparlisib), duvelisib, idelalisib, an AKT inhibitor, an HER2 inhibitor (e.g., lapatinib), herceptin, a taxane (e.g., docetaxel, paclitaxel or nab-paclitaxel), an ADC comprising auristatin, an ADC comprising PBD (e.g., rovalpituzumab tesirine), an ADC comprising maytansinoid (e.g., TDM1), a TRAIL agonist, a proteasome inhibitor (e.g., bortezomib), and a nicotinamide phosphoribosyltransferase (NAMPT) inhibitor; a TNF antagonist (e.g., a soluble fragment of a TNF receptor, such as a p55 or p75 human TNF receptor or a derivative thereof, such as 75kD TNFR-IgG (75kD TNF receptor-IgG fusion protein, ENBREL)); a TNF enzyme antagonist, such as a TNF convertase inhibitor; a muscarinic receptor antagonist; a TGF-β antagonist; an interferon γ; pirfenidone; a chemotherapeutic agent, such as leflunomide or sirolimus or an analog thereof, such as CCI-779; inhibitors of COX2 and cPLA2; NSAID; an immunomodulator; a p38 inhibitor, inhibitors of TPL-2, MK-2 and NFkB, and the like.

In some embodiments, the antibody or the antigen-binding fragment of the present invention can be used with an immune checkpoint inhibitor. In some embodiments, the antibody or the antigen-binding fragment of the present invention is administered in combination with other therapeutic or prophylactic regimens, such as radiotherapy.

### Pharmaceutical composition

The antibody or their derivatives, fragments, analogues, homologues described herein can be incorporated into pharmaceutical compositions suitable for administration. The principles and considerations involved in preparing such compositions and guidelines in the choice of components are well known in the art.

Such compositions typically comprise the antibody or the antigen-binding fragment and a pharmaceutically acceptable carrier. In some embodiments, the antigen-binding fragment is used as the smallest inhibitory fragment that specifically binds to the binding domain of the target protein. For example, a peptide that is based on the variable region sequence of an antibody and retains the ability to bind to a target protein sequence is used. In some embodiments, the pharmaceutical composition further comprises an anti-cancer agent (e.g., an immune checkpoint inhibitor).

In some embodiments, the term "pharmaceutically acceptable" refers to a substance approved by a government regulatory agency or listed in commonly-recognized pharmacopeias for use in animals, particularly in humans. In addition, the "pharmaceutically acceptable carrier" generally refers to a non-toxic solid, semi-solid or liquid filler, diluent, encapsulating material or formulation auxiliary of any type.

The term "carrier" refers to a diluent, an adjuvant, an excipient or a carrier that can be administered to a patient together with the active ingredient. Such carriers can be sterile liquids, such as water and oils, including oils originating from petroleum, animal, plant or synthesis, such as peanut oil, soybean oil, mineral oil, sesame oil, etc. Water is a preferred carrier when the pharmaceutical composition is administered intravenously. A saline aqueous solution, a glucose aqueous solution, and a glycerol solution can also be used as a liquid carrier, especially for injection. Suitable pharmaceutical excipients include starch, glucose, lactose, sucrose, gelatin, malt, rice, flour, chalk, silica gel, sodium stearate, glycerol monostearate, talc, sodium chloride, skimmed milk powder, glycerol, propylene, glycol, water, ethanol, and the like. If desired, the composition may also comprise a small amount of a wetting agent, an emulsifier, or a pH buffering agent such as acetate, citrate or phosphate. Antibacterials such as benzyl alcohol or methylparaben, antioxidants such as ascorbic acid or sodium bisulfite, chelating agents such as ethylenediamine tetraacetic acid, and tonicity adjusting agents such as sodium chloride or dextrose are also contemplated. Such compositions may be in the form of solutions, suspensions, emulsions, tablets, pills, capsules, pulvises, sustained-release formulations and the like. The composition may be formulated as a suppository using conventional binders and carriers such as triglycerides. Oral formulations may comprise standard carriers such as mannitol, lactose, starch, magnesium stearate, sodium saccharin, cellulose and magnesium carbonate of pharmaceutical grade. Examples of suitable pharmaceutical carriers are described in Remington's Pharmaceutical Sciences by E. W. Martin, which is hereby incorporated herein by reference. Such compositions will contain a clinically effective dose of the antibody or the antigen-binding fragment, preferably in a purified form, together with an appropriate amount of a carrier, to provide a form suitable for administration to a patient. The formulation should be suitable for the administration mode. The formulation may be encapsulated in an ampoule bottle, a disposable syringe, or a multi-dose vial made of glass or plastic.

In some embodiments, the composition is formulated into a pharmaceutical composition (e.g., a pharmaceutical composition suitable for intravenous injection into a human being) according to conventional procedures. A composition for intravenous administration is usually a solution in sterile isotonic aqueous buffer. The composition may also comprise a solubilizer and a local anesthetic such as lidocaine to alleviate the pain at the injection site. The pharmaceutical compositions are formulated in dosage unit form for ease of administration and uniformity of dosage. As used herein, dosage unit form refers to physically separable units that are suitable as unitary dosages for a patient to be treated; each unit contains a predetermined amount of one or more of the antibodies calculated to produce the desired therapeutic effect in association with the required pharmaceutical carrier. In general, the active ingredients are provided in a unit dosage form individually or as a mixture. For example, the active ingredients are encapsulated in sealed containers (such as ampoule bottles or sachets) that can indicate the amount of the active agent, in the form of lyophilized powder or anhydrous concentrate. Where the composition is administered by infusion, the composition can be dispensed in infusion bottles containing sterile water or saline of pharmaceutical grade. Where the composition is administrated by injection, an ampoule bottle containing sterile water or saline for injection can be used, so that the active ingredients can be mixed before administration.

In some embodiments, the pharmaceutical composition to be used for *in-vivo* administration is sterile. This can be readily achieved by filtration through sterile filtration membranes.

The pharmaceutical compositions are generally compatible with their intended route of administration. Examples of the routes of administration include parenteral administration, such as intravenous, intradermal, subcutaneous, oral (e.g., inhalational), transdermal (i.e., topical), transmucosal and rectal administration. The pharmaceutical compositions can comprise one or more of the following components: sterile diluents for injection, such as water, saline solutions, fixed oils, polyethylene glycols, glycerol, propylene glycol or other synthetic solvents; antibacterial agents, such as benzyl alcohol or methylparaben; antioxidants, such as ascorbic acid or sodium bisulfite; chelating agents, such as ethylenediamine tetraacetic acid (EDTA); buffers, such as histidine hydrochloride, acetates, citrates or phosphates; tonicity-adjusting agents, such as sodium chloride or dextrose; stabilizers, such as arginine, methionine, trehalose, sucrose or sorbitol; and surfactants, such as tween 20 or tween 80. The pH can be adjusted with acids or bases, such as hydrochloric acid or sodium hydroxide. The pharmaceutical compositions can be packaged in ampoules, disposable syringes or multiple-dose vials made of glass or plastic. In some embodiments, the pharmaceutical compositions suitable for injection include sterile aqueous solutions or dispersions and sterile powders for the instant preparation of sterile injectable solutions or dispersions. In use, the compositions must be sterile and should be fluid to the extent that easy syringability exists. The composition must be stable under the conditions of manufacture and storage and must be able to prevent the contamination of microorganisms such as bacteria and fungi. Prolonged absorption of the injectable compositions can be brought about by including in the composition an agent which delays absorption, for example, aluminum monostearate and gelatin.

For transmucosal or transdermal administration, penetrants appropriate for the barrier to be permeated are used in the formulation. Such penetrants are generally known in the art, and include, for example, detergents, bile salts and fusidic acid derivatives for transmucosal administration. Transmucosal administration can be accomplished through the use of nasal sprays or suppositories. For transdermal administration, one or more of the antibodies may be formulated into ointments, salves, gels, or creams as commonly known in the art.

The pharmaceutical compositions can be placed in a container or dispenser and packaged together with instructions for administration.

The pharmaceutical compositions described herein can also comprise other active ingredients, preferably those with complementary activities that do not adversely affect each other, depending on the particular condition to be treated. In some embodiments, the compositions can comprise an agent that enhances its function, such as a cytotoxic agent, a cytokine, a chemotherapeutic agent or a growth inhibitory agent. Such active ingredients are suitably present in combination in amounts that are effective for the intended purpose.

The composition of the present invention may be formulated in a neutral or salt form. Pharmaceutically acceptable salts include salts formed with anions derived from acids such as hydrochloric acid, phosphoric acid, acetic acid, oxalic acid, tartaric acid, etc., and salts formed with cations derived from, e.g., sodium, potassium, ammonium, calcium, iron hydroxide, isopropylamine, triethylamine, 2-ethylaminoethanol, histidine, procaine, etc.

### Preparation method

Anti-B7-H3 antibodies can be produced by immunizing an animal with, e.g., membrane-bound and/or soluble B7-H3 (e.g., human B7-H3 or immunogenic fragments, derivatives or variants thereof). Alternatively, monoclonal antibodies can be prepared using, e.g., hybridoma methods. In the hybridoma method, a mouse, a hamster or other appropriate host animal, is typically immunized with an immunizing agent to elicit production of lymphocytes or production of antibodies that can specifically bind to the immunizing agent. Alternatively, the lymphocytes can be immunized *in vitro* to generate antibodies.

The immunizing agent used in the hybridoma method generally includes a protein antigen, a fragment thereof, or a fusion protein thereof (e.g., hB7-H3-Fc, the amino acid sequence of which is set forth in SEQ ID NO: 1). Generally, if a human cell is desired, a peripheral blood lymphocyte is used, or if a cell of non-human mammalian origin is desired, a spleen cell or lymph node cell is used. The lymphocyte is then fused with an immortalized cell line using a suitable fusing agent, such as polyethylene glycol, to form a hybridoma cell. Immortalized cell lines are usually rat or mouse myeloma cell lines. The hybridoma cell may be cultured in a suitable culture medium preferably containing one or more substances that inhibit the growth or survival of unfused immortalized cells. For example, if the parent cells lack hypoxanthine-guanine phosphoribosyltransferase (HGPRT or HPRT), the culture medium for the hybridoma generally contains hypoxanthine, aminopterin and thymine ("HAT medium"), which prevent the growth of HGPRT-deficient cells.

The monoclonal antibody can also be prepared by recombinant DNA methods, such as those described in U.S. Pat. No. 4,816,567. DNA encoding the monoclonal antibody described herein can be isolated and sequenced using conventional methods (e.g., by using oligonucleotide probes that can specifically bind to genes encoding the heavy and light chains of murine antibodies). Hybridoma cells may serve as a source of such an DNA. Once isolated, the DNA can be placed into expression vectors, which are then transfected into a host cell such as a Chinese hamster ovary cell (CHO cell) , a human embryonic kidney 293 cell (HEK293 cell), a simian COS cell, a PER. NS0 cell, a SP2/0 cell, a YB2/0 cell or a myeloma cell that does not otherwise produce immunoglobulins, thereby obtaining a synthetic monoclonal antibody in the recombinant host cell. The DNA can also be modified, for example, by substituting the coding sequence for human heavy and light chain constant domains for the homologous murine sequences (see U.S. Pat. No. 4,816,567) or by covalently linking the immunoglobulin coding sequence to all or part of the coding sequence for a non-immunoglobulin polypeptide. Such a non-immunoglobulin polypeptide can be substituted for the constant domains of the antibodies described herein, or can be substituted for the variable domains of one antigen-combining site of the antibodies described herein to produce chimeric bivalent antibodies.

For single-chain Fvs (scFvs), reference can be made to the technology for the production of single-chain units (U.S. Pat. No. 4,694,778). Single-chain units are formed by linking the heavy and light chain fragments of the Fv region via an amino acid bridge, resulting in a single-chain fusion peptide. Techniques for the assembly of functional Fv fragments in *E. coli* may also be used. Examples of techniques that may be used to produce scFvs and antibodies include those described in U.S. Pat. Nos. 4,946,778 and 5,258,498.

In addition, another efficient method for producing recombinant antibodies, which in particular is capable of producing primate antibodies comprising monkey variable region and human constant region sequences, is disclosed in U.S. Pat. Nos. 5,658,570, 5,693,780 and 5,756,096, each of which is incorporated herein by reference in its entirety.

In some embodiments, the antibody does not elicit a deleterious immune response in the treated animal (e.g., human being). In some embodiments, the antibody, the antigen-binding fragment or the derivative thereof disclosed herein is modified using techniques well known in the art to reduce the immunogenicity. For example, the antibody can be humanized, primatized or deimmunized, or a chimeric antibody can be prepared. Such antibodies are derived from non-human antibodies, typically murine or primate antibodies, which retain or substantially retain the antigen-binding properties of the parent antibody but are less immunogenic in humans. This can be accomplished by a variety of methods, including: (a) grafting the entire variable region of a non-human origin to the constant region of a human origin to produce a chimeric antibody, wherein methods for producing chimeric antibodies are known in the art, see U.S. Pat. Nos. 5,807,715, 4,816,567 and 4,816,397, the entire contents of which are incorporated herein by reference; (b) grafting at least a portion of one or more non-human complementarity determining regions (CDRs) to the framework region and constant region of a human origin, with or without retention of critical framework residues; or (c) grafting the entire variable region of a non-human origin, but "hiding" the surface residues by replacing them with portions of a human-like origin. Generally, framework residues in the human framework regions will be substituted by corresponding residues from the CDR donor antibody, preferably residues that may improve the antigen binding. Such framework substitutions can be identified by methods well known in the art, for example, by modeling the interaction of the CDR and framework residues to identify framework residues that play an important role in antigen binding and by sequence alignment to identify abnormal framework residues at particular positions (see U.S. Pat. No. 5,585,089, the content of which is incorporated herein by reference in its entirety). Antibodies can be humanized using a variety of techniques well known in the art, such as CDR grafting (EP 239,400; WO 91/09967; U.S. Pat. Nos. 5,225,539, 5,530,101 and 5,585,089), repair or surface rearrangement (EP 592,106; EP 519,596), and chain rearrangement (U.S. Pat. No. 5,565,332), the contents of which are incorporated herein by reference in their entireties.

Deimmunization may also be used to reduce the immunogenicity of the antibody. In the present invention, the term "deimmunization" includes the alteration of the antibody to modify T cell epitopes (see, e.g., Pat. Nos. WO/9852976 A1 and WO/0034317 A2). For example, a heavy chain variable region sequence and a light chain variable region sequence from the original antibody are analyzed, and a "map" of human T cell epitopes from each variable region is generated, showing the positions of the epitopes relative to the complementarity determining regions (CDRs) and other critical residues within the sequence. Individual T cell epitopes from the T cell epitope map are analyzed to identify alternative amino acid substitutions with a lower risk of altering antibody activity. A series of alternative heavy chain variable region sequences and light chain variable region sequences comprising combinations of amino acid substitutions are designed and subsequently incorporated into a series of binding polypeptides. The genes of intact heavy and light chains comprising the modified variable regions and human constant regions are cloned into an expression vector, and the plasmid is then transferred into a cell line to produce an intact antibody. The antibodies are compared in appropriate biochemical and biological experiments to identify the optimal antibody.

The antibodies can be prepared by a variety of methods known in the art, including phage display methods using antibody libraries from immunoglobulin sequences. Reference may also be made to U.S. Pat. Nos. 4,444,887 and 4,716,111, and PCT Publication Nos. WO98/46645, WO98/50433, WO98/24893, WO98/16654, WO96/34096, WO96/33735 and WO91/10741, each of which is incorporated herein by reference in its entirety.

Fully human antibodies that recognize selective epitopes can be produced using a technique known as "guided selection". In this method, a selected non-human monoclonal antibody such as a mouse antibody is used to guide the screening of fully human antibodies that recognize the same epitope (see U.S. Pat. No. 5,565,332, which is incorporated herein by reference in its entirety).

In another embodiment, DNA encoding the desired monoclonal antibody can be isolated and sequenced using conventional methods (e.g., using oligonucleotide probes that can specifically bind to genes encoding the heavy and light chains of a murine antibody). Isolated and subcloned hybridoma cells can be preferred sources of such DNA. Once isolated, the DNA can be inserted into an expression vector and then transfected into prokaryotic or eukaryotic host cells (such as *E*. *coli* cells, simian COS cells, Chinese hamster ovary cells (CHO cells), or myeloma cells that do not produce other immunoglobulins). The isolated or synthetic DNA can also be used to prepare sequences of the constant and variable regions of an antibody, as described in U.S. Pat. No. 5,658,570, the content of which is incorporated herein by reference in its entirety. This method extracts RNA from the selected cells and conversion to cDNA, followed by amplification by PCR using Ig-specific primers. Suitable probes for this purpose are also described in U.S. Pat. No. 5,658,570.

In addition, using conventional recombinant DNA techniques, one or more CDRs of the antibody of the present invention can be inserted into framework regions, e.g., into human framework regions to construct a humanized non-fully human antibody. The framework regions may be naturally occurring or consensus framework regions, preferably human framework regions. Some polynucleotides may encode antibodies produced by the framework region and CDR combinations that specifically bind to at least one epitope of an target antigen. One or more amino acid substitutions may be made within the framework regions, and amino acid substitutions capable of improving the binding of the antibody to the antigen thereof may be selected. Additionally, substitution or deletion of one or more cysteine residues in the variable regions involved in interchain disulfide bond formation can be made in this manner, thereby producing an antibody molecule lacking one or more interchain disulfide bonds. Other alterations to the polynucleotides made within the technology scope of the art are also encompassed by the present invention. Antibodies can be prepared by using conventional recombinant DNA techniques. Vectors and cell lines for antibody production can be selected, constructed and cultured using techniques well known to those skilled in the art. These techniques are described in various laboratory manuals and main publications.

In some embodiments, the anti-B7-H3 antibody or the antigen-binding fragment of the present invention is subjected to glycosylation. For example, deglycosylated antibodies (i.e., the antibodies lack glycosylation) can be prepared. Glycosylation can be altered, for example, to increase the affinity of an antibody for an antigen. Such modifications can be accomplished, for example, by altering one or more glycosylation sites within the antibody sequence. For example, one or more amino acid substitutions may be made to eliminate one or more glycosylation sites in the variable region, thereby eliminating glycosylation at those sites. Such deglycosylation can increase the affinity of an antibody for an antigen. Such methods are further described in detail in PCT publication WO 2003016466 A2 and U.S. Pat. Nos. 5,714,350 and 6,350,861, each of which is incorporated herein by reference in its entirety.

In addition, modified anti-B7-H3 antibodies or antigen-binding fragments with altered glycosylation types, such as low-fucosylated antibodies or antibodies with increased bisecting GlcNAc structures, can be prepared. These changes have been shown to increase the ADCC ability of the antibody. Such modifications can be achieved, for example, by expressing the antibody in a host cell with altered glycosylation mechanisms. Cells with altered glycosylation mechanisms have been described in the art and can be used as host cells for expressing recombinant antibodies of the present invention and thereby producing antibodies with altered glycosylation. See, e.g., European Patent No.: EP 1,176,195 and PCT publications WO 03/035835 and WO 99/5434280, each of which is incorporated herein by reference in its entirety.

Antibodies of the present invention (e.g., anti-B7-H3 antibodies) can be prepared by recombinant expression of the heavy and light chain genes of the antibody in a host cell. For example, a host cell is transfected with one or more recombinant expression vectors carrying heavy and light chain DNA segments encoding the antibody such that the heavy and light chains are expressed in the host cell, the expressed antibody can be secreted into the medium in which the host cell is cultured, and the antibody can be isolated from the medium. "Transfect" refers to a wide variety of techniques commonly used to introduce a foreign DNA into eukaryotic host cells, such as electroporation, lipofection, calcium phosphate precipitation, DEAE-dextran transfection, and the like. Standard recombinant DNA methods for obtaining antibody heavy and light chain genes, incorporating these genes into expression vectors and introducing the vectors into host cells are well known in the art, e.g., those methods described in U.S. Pat. No. 4,816,397. The DNAs expressing the heavy and light chains of the antibody may be placed in the same vector or placed in different vectors; if placed in different vectors, the vectors expressing the heavy and light chains of the antibody may transfect host cells in an appropriate ratio. In some embodiments, the antibody expression vector includes at least one promoter element, an antibody coding sequence, a transcription termination signal, and a polyA tail. Other elements may include enhancers, Kozak sequences (GCCACC, set forth in SEQ ID NO: 53), and intervening sequences flanked by donor and acceptor sites for RNA splicing. Efficient transcription can be obtained by early and late promoters of SV40, long terminal repeats from retroviruses such as RSV, HTLV1 and HIVI, and early promoters of cytomegalovirus, and promoters from other cells such as actin promoter can also be used. Suitable expression vectors may include pIRES1neo, pRetro-Off, pRetro-On, pLXSN, pLNCX, pcDNA3.1(+/-), pcDNA/Zeo(+/-), pcDNA3.1/Hygro(+/-), pSVL, pMSG, pRSVcat, pSV2dhfr, pBC12MI, pCS2, pCHO1.0 or the like. Commonly used mammalian cells (host cells) include HEK293 cells (e.g., HEK293F cells), Cos1 cells, Cos7 cells, CV1 cells, murine L cells, CHO cells, and the like.

To express an antibody of the present invention (e.g., an anti-B7-H3 antibody), the DNA encoding the full-length light and heavy chains can be inserted into an expression vector such that the genes are operably linked to transcriptional and translational control sequences. "Operably linked" means that the antibody genes are linked into a vector such that transcriptional and translational control sequences within the vector perform their intended functions of regulating the transcription and translation of the antibody genes.

Antibody (e.g., anti-B7-H3 antibody) genes can be inserted into an expression vector by standard methods (e.g., linking the antibody gene fragment to complementary restriction sites on the vector, or blunt end ligation if no restriction sites are present). The expression vector may already carry antibody constant region sequences prior to insertion of the antibody-related light or heavy chain gene sequences. For example, one method for converting the anti-B7-H3 antibody-related VH and VL sequences into full-length antibody genes is to insert them into an expression vector that already encodes a heavy chain constant region and a light chain constant region, such that the VH segment is operably linked to the CH segment within the vector and the VL segment is operably linked to the CL segment within the vector. Alternatively, the recombinant expression vector may encode a signal peptide that facilitates secretion of the heavy and light chains of an antibody from a host cell. Alternatively, the antibody heavy and light chain genes may be cloned into a vector encoding a signal peptide that facilitates secretion of the antibody heavy and light chains from a host cell, such that the signal peptide is linked in frame to the amino termini of the antibody heavy and light chain genes. The signal peptide may be an immunoglobulin signal peptide or a heterologous signal peptide (i.e., a signal peptide from a non-immunoglobulin protein), for example, MEFGLSWVFLVAILKGVQC (SEQ ID NO: 45), MKHLWFFLLLVAAPRWVLS (SEQ ID NO: 46), MDMRVLAQLLGLLLLCFPGARC (SEQ ID NO: 47) or MVLQTQVFISLLLWISGAYG (SEQ ID NO:48).

In addition to the antibody heavy and light chain genes, the recombinant expression vector may also carry regulatory sequences that control expression of the antibody chain genes in the host cell. Regulatory sequences include promoters, enhancers and other expression control elements (e.g., polyadenylation signals) that control the transcription or translation of antibody chain genes. Those skilled in the art will understand that the design of an expression vector, including the choice of regulatory sequences, may depend on such factors as the choice of host cell to be transformed, the level of expression of the desired protein, and the like. Suitable regulatory sequences for expression in mammalian host cells include viral elements that direct high-level protein expression in mammalian cells, such as promoters and/or enhancers derived from cytomegalovirus (CMV) (e.g., the CMV promoter/enhancer), simian virus 40 (SV40) (e.g., the SV40 promoter/enhancer), adenovirus (e.g., the adenovirus major late promoter (AdMI,P)), and polyoma. For further description of viral regulatory elements and sequences thereof, see, e.g., U.S. Pat. Nos. 5,168,062, 4,510,245 and 4,968,615.

In addition to the antibody chain genes and regulatory sequences, the recombinant expression vector may carry other sequences, such as a sequence that regulates replication of the vector in a host cell (e.g., an origin of replication) and a selectable marker gene. The selectable marker gene facilitates selection of a host cell into which the vector has been introduced (see, e.g., U.S. Pat. Nos. 4,399,216, 4,634,665 and 5,179,017). For example, typically a marker gene that imparts resistance to drugs (such as G418, hygromycin or methotrexate) to a host cell into which the vector has been introduced can be selected. Suitable selectable marker genes include the dihydrofolate reductase (DHFR) gene (for use in DHFR-host cells with methotrexate selection/amplification), the neo gene (for G418 selection), and the GS gene. For expression of heavy and light chains, an expression vector encoding the heavy and light chains is transfected into a host cell by standard techniques.

Antibodies of the present invention (e.g., anti-B7-H3 antibodies) can be expressed in eukaryotic host cells. In certain embodiments, expression of an antibody is performed in eukaryotic cells, such as mammalian host cells. Exemplary host cells for expressing the antibodies of the present invention include Chinese hamster ovary cells (CHO cells) or CHO-S, CHO-dhfr-, CHO/DG44 or ExpiCHO obtained by modification of CHO cells, NSO myeloma cells, COS cells, SP2 cells, CV1 cells, murine L cells, human embryonic kidney HEK293 cells, or HEK293T, HEK293F or HEK293E cells obtained by modification of HEK293 cells. After a recombinant expression vector encoding an antibody chain gene is introduced into a host cell, the host cell is cultured in a medium for a period of time sufficient to allow the antibody to be expressed in the host cell or allow the antibody to be secreted into the medium to produce the antibody. The antibody can be isolated from the medium using standard protein purification methods.

For recombinant expression of an antibody of the present invention (e.g., anti-B7-H3 antibody), a host cell can be co-transfected with two recombinant expression vectors, a first recombinant expression vector encoding the heavy chain of the antibody and a second recombinant expression vector encoding the light chain of the antibody. The two recombinant expression vectors may contain the same selectable marker, or they may each contain separate selectable markers. Alternatively, a host cell may be transfected with a recombinant expression vector encoding the heavy and light chains of the antibody.

Antibodies of the present invention (e.g., anti-B7-H3 antibodies) can also be produced by chemical synthesis. Variant antibodies can also be generated using a cell-free platform.

The antibody of the present invention (e.g., anti-B7-H3 antibody) produced by recombinant expression can be purified by any method known in the art for purifying immunoglobulin molecules, for example, by chromatography (e.g., ion exchange, affinity, and sizing column chromatography), centrifugation, differential solubility, or any other standard technique for purifying proteins. For example, protein A or protein G is subjected to affinity chromatography, which provides primarily the IgG fraction in immune serum. In addition, the specific antigen targeted by the immunoglobulin, or an epitope thereof, can be immobilized on a column to purify the immune specific antibody by immunoaffinity chromatography. The antibody of the present invention (e.g., anti-B7-H3 antibody) can be fused to heterologous polypeptide sequences known in the art to facilitate purification. For purification of immunoglobulins, reference can be made to the D. Wilkinson's article (The Scientist, published by the Scientist, Inc., Philadelphia Pa., Vol. 14, No. 8 (Apr. 17, 2000), pp. 25-28).

In addition, mutations can be introduced in the nucleotide sequence encoding the antibody of the present invention using standard techniques known to those skilled in the art, including but not limited to site-directed mutations resulting in amino acid substitutions and PCR-mediated mutations. The variant (including derivative) encodes a substitution of less than 50 amino acids, a substitution of less than 40 amino acids, a substitution of less than 30 amino acids, a substitution of less than 25 amino acids, a substitution of less than 20 amino acids, a substitution of less than 15 amino acids, a substitution of less than 10 amino acids, a substitution of less than 5 amino acids, a substitution of less than 4 amino acids, a substitution of less than 3 amino acids or a substitution of less than 2 amino acids relative to the VH CDR1, VH CDR2 and VH CDR3 of the original heavy chain variable region and the VL CDR1, VL CDR2 or VL CDR3 of the original light chain variable region. Alternatively, mutations can be introduced randomly along all or part of the encoding sequence, for example by saturation mutagenesis, and the resulting mutants can be screened for the biological activity to identify mutants that retain the activity.

In some embodiments, the substitutions described herein are conservative amino acid substitutions. In some embodiments, the gene sequence encoding the heavy chain of antibody V3 is set forth in SEQ ID NO: 49, wherein the underlined parts encode the VH CDRs; the heavy chain amino acid sequence of antibody V3 is set forth in SEQ ID NO: 50, wherein the underlined parts are the VH CDRs.
SEQ ID NO: 49 is shown below: 
GAGGTGCAGCTGGTGCAGAGCGGCGCCGAGGTGAAGAAGAGCGGCGAGTCCCTGA
AGATCTCCTGTAAGGCTAGCGGCTATACCTTTACCGATTACGATATCAACTGGGTGCGG
CAGATGCCTGGCAAGGGCCTGGAGTGGATCGGCTGGATCTTTCCCGGCGATGACACC
ACCAAGTACAACGAGAAGTTCAAGGGCCAGGTGACCCTGAGCGCTGATAAGTCCAC
CAACACCGCCTACATGCAGTGGTCCTCCCTGAAGGCCTCCGACACCGCCATGTATTAT
TGCGCCCGGTCCCCCAGCTTCGACTACTGGGGCCAGGGTACCCTGGTTACCGTTAGC
AGCGCGAGCACCAAAGGCCCGAGCGTGTTTCCGCTGGCCCCGAGCAGCAAAAGCAC
CAGCGGTGGCACCGCAGCGCTGGGTTGCCTGGTGAAAGATTATTTCCCGGAACCGGT
GACGGTGTCGTGGAACTCAGGCGCCCTGACCAGCGGCGTGCACACCTTCCCGGCTGT
CCTACAGTCCTCAGGACTCTACTCCCTCAGCAGCGTGGTGACCGTGCCCTCCAGCAG
CTTGGGCACCCAGACCTACATCTGCAACGTGAATCACAAGCCCAGCAACACCAAGGT
GGACAAGAAAGTTGAGCCCAAATCTTGTGACAAAACTCACACATGCCCACCGTGCCC
AGCACCTGAACTCCTGGGGGGACCGTCAGTCTTCCTCTTCCCCCCAAAACCCAAGGA
CACCCTCATGATCTCCCGGACCCCTGAGGTCACATGCGTGGTGGTGGACGTGAGCCA
CGAAGACCCTGAGGTCAAGTTCAACTGGTACGTGGACGGCGTGGAGGTGCATAATGC
CAAGACAAAGCCGCGGGAGGAGCAGTACAACAGCACGTACCGTGTGGTCAGCGTCC
TCACCGTCCTGCACCAGGACTGGCTGAATGGCAAGGAGTACAAGTGCAAGGTCTCC
AACAAAGCCCTCCCAGCCCCCATCGAGAAAACCATCTCCAAAGCCAAAGGGCAGCC
CCGAGAACCACAGGTGTACACCCTGCCCCCATCCCGGGATGAGCTGACCAAGAACC
AGGTCAGCCTGACCTGCCTGGTCAAAGGCTTCTATCCCAGCGACATCGCCGTGGAGT
GGGAGAGCAATGGGCAGCCGGAGAACAACTACAAGACCACGCCTCCCGTGCTGGAC
TCCGACGGCTCCTTCTTCCTCTACAGCAAGCTCACCGTGGACAAGAGCAGGTGGCAG
CAGGGGAACGTCTTCTCATGCTCCGTGATGCATGAGGCTCTGCACAACCACTACACG
CAGAAGAGCCTCTCCCTGTCTCCGGGTAAA
SEQ ID NO: 50 is shown below:

EVQLVQSGAEVKKSGESLKISCKASGYTFTDYDINWVRQMPGKGLEWIGWIFPGDDTTK
YNEKFKGQVTLSADKSTNTAYMQWSSLKASDTAMYYCARSPSFDYWGQGTLVTVSSAS
TKGPSVFPLAPSSKSTSGGTAALGCLVKDYFPEPVTVSWNSGALTSGVHTFPAVLQSSGL
YSLSSVVTVPSSSLGTQTYICNVNHKPSNTKVDKKVEPKSCDKTHTCPPCPAPELLGGPS
VFLFPPKPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTLPPSRD
ELTKNQVSLTCLVKGFYPSDIAVEWESNGQPENNYKTTPPVLDSDGSFFLYSKLTVDKSR
WQQGNVFSCSVMHEALHNHYTQKSLSLSPGK

In some embodiments, the gene sequence encoding the light chain of antibody V3 is set forth in SEQ ID NO: 51, wherein the underlined parts encode the VL CDRs; the light chain amino acid sequence of antibody V3 is set forth in SEQ ID NO: 52, wherein the underlined parts are the VL CDRs.
SEQ ID NO: 51 is shown below:

CAGATCGTGCTGACCCAGAGCCCCGGCACCCTGTCCCTGAGCCCTGGAGAGAGGGC
CACCCTGAGCTGTAGCGCTAGCTCCACCATCGGCTTTATGTATTGGTATCAGCAGAAG
CCTGGCCAGGCCCCCCGGAGATGGATCTACGACACCAGCAAGCTGGCCAGCGGCGT
GCCTGACAGGTTTTCCGGCAGCGGCTCCGGCACCGACTATACCCTGACCATCAGCAG
GCTGGAGCCCGAGGATTTCGCCGTGTATTATTGCCACCAGCGGAGCAGCTATCCTACC
TTTGGCCAGGGCACCAAGGTGGAGATCAAGCGTACGGTGGCTGCACCATCTGTCTTC
ATCTTCCCGCCATCTGATGAGCAGTTGAAATCTGGAACTGCCTCTGTTGTGTGCCTGC
TGAATAACTTCTATCCCAGAGAGGCCAAAGTACAGTGGAAGGTGGATAACGCCCTCC
AATCGGGTAACTCCCAGGAGAGTGTCACAGAGCAGGACAGCAAGGACAGCACCTAC

AGCCTCAGCAGCACCCTGACGCTGAGCAAAGCAGACTACGAGAAACACAAAGTCTA
CGCCTGCGAAGTCACCCATCAGGGCCTGAGCTCGCCCGTCACAAAGAGCTTCAACA
GGGGAGAGTGT
SEQ ID NO: 52 is shown below:
QIVLTQSPGTLSLSPGERATLSCSASSTIGFMYWYQQKPGQAPRRWIYDTSKLASGVPDR
FSGSGSGTDYTLTISRLEPEDFAVYYCHQRSSYPTFGQGTKVEIKRTVAAPSVFIFPPSDEQ
LKSGTASVVCLLNNFYPREAKVQWKVDNALQSGNSQESVTEQDSKDSTYSLSSTLTLSK
ADYEKHKVYACEVTHQGLSSPVTKSFNRGEC

### Examples

The technical solution of the present invention will be further illustrated below through specific examples, which are not intended to limit the protection scope of the present invention. Some nonessential modifications or adjustments made by other people according to the concept of the present invention still fall within the protection scope of the present invention.

The materials, reagents, etc. used in the following examples can be commercially available or obtained using known methods unless otherwise stated.

Procedures for constructing VISTA-CHO cells: a gene sequence encoding VISTA (NCBI Reference Sequence: NP_071436.1) is inserted into an expression vector, which is then stably transfected into CHO cells, thus obtaining VISTA-CHO cells.

Procedures for constructing Tim3-CHO cells: a gene sequence encoding Tim3 (NCBI Reference Sequence: NP_116171.3) is inserted into an expression vector, which is then stably transfected into CHO cells, thus obtaining Tim3-CHO cells.

Procedures for constructing Lag3-CHO cells: a gene sequence encoding Lag3 (NCBI Reference Sequence: NP_002277.4) is inserted into an expression vector, which is then stably transfected into CHO cells, thus obtaining Lag3-CHO cells.

### Example 1. Preparation of Antigens

Preparation of antigen hB7-H3-Fc: the gene sequence encoding the antigen hB7-H3-Fc (the amino acid sequence of the antigen hB7-H3-Fc is set forth in SEQ ID NO: 1 and is constructed by adding Fc (set forth in SEQ ID NO: 3) of IgG1 to the C-terminus of the human B7-H3 extracellular domain (set forth in SEQ ID NO: 2)) was cloned into an expression vector, which was then transiently transfected into HEK293F cells, and the antigen hB7-H3-Fc was obtained after Protein A affinity chromatography purification.

Preparation of antigen hB7-H3-His: the gene sequence encoding the antigen hB7-H3-His (the amino acid sequence of the antigen hB7-H3-His is set forth in SEQ ID NO: 4 and is constructed by adding 10× HIS tag (HHHHHHHHHH, set forth in SEQ ID NO: 5) to the C-terminus of the human B7-H3 extracellular domain (set forth in SEQ ID NO: 2)) was cloned into an expression vector, which was then transiently transfected into HEK293F cells, and the antigen hB7-H3-His was obtained after purification with a nickel column.

Amino acid sequence of antigen hB7-H3-Fc:

MLRRRGSPGMGVHVGAALGALWFCLTGALEVQVPEDPVVALVGTDATLCCSFSPEPGF
SLAQLNLIWQLTDTKQLVHSFAEGQDQGSAYANRTALFPDLLAQGNASLRLQRVRVAD
EGSFTCFVSIRDFGSAAVSLQVAAPYSKPSMTLEPNKDLRPGDTVTITCSSYQGYPEAEV
FWQDGQGVPLTGNVTTSQMANEQGLFDVHSILRVVLGANGTYSCLVRNPVLQQDAHS
SVTITPQRSPTGAVEVQVPEDPVVALVGTDATLRCSFSPEPGFSLAQLNLIWQLTDTKQL
VHSFTEGRDQGSAYANRTALFPDLLAQGNASLRLQRVRVADEGSFTCFVSIRDFGSAAV
SLQVAAPYSKPSMTLEPNKDLRPGDTVTITCSSYRGYPEAEVFWQDGQGVPLTGNVTTS
QMANEQGLFDVHSVLRVVLGANGTYSCLVRNPVLQQDAHGSVTITGQPMTDKTHTCPP

CPAPELLGGPSVFLFPPKPKDTLMISRTPEVTCVVVDVSHEDPEVKFNWYVDGVEVHNA
KTKPREEQYNSTYRVVSVLTVLHQDWLNGKEYKCKVSNKALPAPIEKTISKAKGQPREP
QVYTLPPSRDELTKNQVSLTCLVKGFYPSDIAVEWESNGQPENNYKTTPPVLDSDGSFFL
YSKLTVDKSRWQQGNVFSCSVMHEALHNHYTQKSLSLSPGK (set forth in SEQ ID NO: 1).

Amino acid sequence of human B7-H3 extracellular domain:

MLRRRGSPGMGVHVGAALGALWFCLTGALEVQVPEDPVVALVGTDATLCCSFSPEPGF
SLAQLNLIWQLTDTKQLVHSFAEGQDQGSAYANRTALFPDLLAQGNASLRLQRVRVAD
EGSFTCFVSIRDFGSAAVSLQVAAPYSKPSMTLEPNKDLRPGDTVTITCSSYQGYPEAEV
FWQDGQGVPLTGNVTTSQMANEQGLFDVHSILRVVLGANGTYSCLVRNPVLQQDAHS
SVTITPQRSPTGAVEVQVPEDPVVALVGTDATLRCSFSPEPGFSLAQLNLIWQLTDTKQL
VHSFTEGRDQGSAYANRTALFPDLLAQGNASLRLQRVRVADEGSFTCFVSIRDFGSAAV
SLQVAAPYSKPSMTLEPNKDLRPGDTVTITCSSYRGYPEAEVFWQDGQGVPLTGNVTTS
QMANEQGLFDVHSVLRVVLGANGTYSCLVRNPVLQQDAHGSVTITGQPMT (set forth in
SEQ ID NO: 2).

Amino acid sequence of Fc of IgG1:

DKTHTCPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTTPP
VLDSDGSFFLYSKLTVDKSRWQQGNVFSCSVMHEALHNHYTQKSLSLSPGK (set forth in
SEQ ID NO: 3).

Amino acid sequence of antigen hB7-H3-His:

MLRRRGSPGMGVHVGAALGALWFCLTGALEVQVPEDPVVALVGTDATLCCSFSPEPGF
SLAQLNLIWQLTDTKQLVHSFAEGQDQGSAYANRTALFPDLLAQGNASLRLQRVRVAD
EGSFTCFVSIRDFGSAAVSLQVAAPYSKPSMTLEPNKDLRPGDTVTITCSSYQGYPEAEV
FWQDGQGVPLTGNVTTSQMANEQGLFDVHSILRVVLGANGTYSCLVRNPVLQQDAHS
SVTITPQRSPTGAVEVQVPEDPVVALVGTDATLRCSFSPEPGFSLAQLNLIWQLTDTKQL
VHSFTEGRDQGSAYANRTALFPDLLAQGNASLRLQRVRVADEGSFTCFVSIRDFGSAAV
SLQVAAPYSKPSMTLEPNKDLRPGDTVTITCSSYRGYPEAEVFWQDGQGVPLTGNVTTS
QMANEQGLFDVHSVLRVVLGANGTYSCLVRNPVLQQDAHGSVTITGQPMTHHHHHH
HHH (set forth in SEQ ID NO: 4).

### Example 2.Chimeric Antibodies

The amino acid sequences of the VH CDRs, VL CDRs, VHs and VLs of the murine antibodies are shown in Tables 1-3.

**Table 1. Amino acid sequences of VH CDRs (Kabat numbering)**

| No. | Type | Amino acid sequence | SEQ ID NO: |
|---|---|---|---|
| MH | VH CDR1 | DYDIN | 6 |
| | VH CDR2 | WIFPGDDTTKYNEKFKG | 7 |
| | VH CDR3 | SPSFDY | 8 |

**Table 2. Amino acid sequences of VL CDRs (Kabat numbering)**

| No. | Type | Amino acid sequence | SEQ ID NO: |
|---|---|---|---|
| ML-1 | VL CDR1 | SASSTIGFMY | 9 |
| ML-2 | | KASQDINRFLS | 10 |
| ML-3 | | RASKSVSTSGYSYMH | 11 |
| ML-4 | | RATGNIHNYLA | 12 |
| ML-5 | | RASKNIYSFLG | 13 |
| ML-1 | VL CDR2 | DTSKLAS | 14 |
| ML-2 | | RANRLRD | 15 |
| ML-3 | | LVSNLES | 16 |
| ML-4 | | SAKTLAD | 17 |
| ML-5 | | NAKTLAE | 18 |
| ML-1 | VL CDR3 | HQRSSYPT | 19 |
| ML-2 | | LQYDEFPPFT | 20 |
| ML-3 | | QHIRELT | 21 |
| ML-4 | | QHFWSIPWT | 22 |
| ML-5 | | QHHYGTPPYT | 23 |

**Table 3. Amino acid sequences of variable regions of murine antibodies (Kabat numbering)**

| No. | Type | Amino acid sequence | SEQ ID NO: |
|---|---|---|---|
| MH | VH | QVQLQQSGPELVKPGASVRISCKASGYTFTDYDINWVQQ RPGQGLEWIGWIFPGDDTTKYNEKFKGRATLTADKSSN TAYMQLNGLTSENSAVYFCARSPSFDYWGQGTLVTVSS | 24 |
| ML-1 | VL | QIVLTQSPAIMSASPGERVTMTCSASSTIGFMYWYQQKP GTSPKRWIYDTSKLASGVPARFSVSGSGTSYSLTISSMEA EDAATYYCHQRSSYPTFGGGTKLEIK | 25 |
| ML-2 | | DIKMTQSPSSMYASLGERVTITCKASQDINRFLSWFQQK PGKSPKTLIYRANRLRDGVPSRFSGNGSGQDYSLTISSLE YEDMGIYYCLQYDEFPPFTFGSGTKLEIK | 26 |
| ML-3 | | DIVLTQSPASLAVSLGQRATISCRASKSVSTSGYSYMHW NQQKPGQPPRLLIYLVSNLESGVPARFSGSGSGTDFTLNI HPVEEEDAATYYCQHIRELTFGAGTKLELK | 27 |
| ML-4 | | DIQMTQSTASLSAFVGETVTITCRATGNIHNYLAWYHQK QGKSPHLLVYSAKTLADGVPSRFSGSGSGTQYSLQINSL HPEDFGTYYCQHFWSIPWTFGGGTKLEIK | 28 |
| ML-5 | | DIQMTQSPASLSASVGETVTITCRASKNIYSFLGWIQQRQ GKSPQSLVYNAKTLAEGVPSRFSGSGSGTQFSLKINSLQP EDFGNYYCQHHYGTPPYTFGGGTKLEIK | 29 |

The assembly scheme of the chimeric antibodies is shown in Table 4, the VH and CH constitute the heavy chain of the antibody, and the VL and CL constitute the light chain of the antibody; the antibodies are indicated by "antibody" + antibody number, e.g., the heavy chain of antibody M1 consists of the VH set forth in SED ID NO: 24 and the CH set forth in SED ID NO: 32, and the light chain of antibody M1 consists of the VL set forth in SED ID NO: 25 and the CL set forth in SED ID NO: 34. The heavy chain of the control antibody M30-H1-L4 is set forth in SEQ ID NO: 30 and its light chain is set forth in SEQ ID NO: 31 (see Table 5). The CDRs of the chimeric antibodies are shown in Table 6.

Gene sequences encoding the heavy chain and the light chain of the antibody were cloned into an expression vector to obtain a recombinant expression vector, which was then transiently transfected into HEK293F cells. After culturing and Protein A affinity chromatography purification, the antibody was obtained and its sequence was consistent with the expected sequence as determined by sequencing.

**Table 4. Number and assembly scheme of chimeric antibodies**

| Antibody No. | VH SEQ ID NO: | CH SEQ ID NO: | VL SEQ ID NO: | CL SEQ ID NO: |
|---|---|---|---|---|
| M1 | 24 | 32 | 25 | 34 |
| M2 | 24 | 32 | 26 | 34 |
| M3 | 24 | 32 | 27 | 34 |
| M4 | 24 | 32 | 28 | 34 |
| M5 | 24 | 32 | 29 | 34 |

**Table 5. Amino acid sequence of control antibody M30-H1-L4**

| No. | Type | Amino acid sequence | SEQ ID NO: |
|---|---|---|---|
| M30-H1-L4 | Heavy chain | QVQLVQSGAEVKKPGSSVKVSCKASGYTFTNYVMHW VRQAPGQGLEWMGYINPYNDDVKYNEKFKGRVTITA DESTSTAYMELSSLRSEDTAVYYCARWGYYGSPLYYFD YWGQGTLVTVSSASTKGPSVFPLAPSSKSTSGGTAALG CLVKDYFPEPVTVSWNSGALTSGVHTFPAVLQSSGLYS LSSVVTVPSSSLGTQTYICNVNHKPSNTKVDKRVEPKS CDKTHTCPPCPAPELLGGPSVFLFPPKPKDTLMISRTPE VTCVVVDVSHEDPEVKFNWYVDGVEVHNAKTKPREE QYNSTYRVVSVLTVLHQDWLNGKEYKCKVSNKALPA PIEKTISKAKGQPREPQVYTLPPSREEMTKNQVSLTCLV KGFYPSDIAVEWESNGQPENNYKTTPPVLDSDGSFFLY SKLTVDKSRWQQGNVFSCSVMHEALHNHYTQKSLSLS PGK | 30 |
| | Light chain | EIVLTQSPATLSLSPGERATLSCRASSRLIYMHWYQQKP GQAPRPLIYATSNLASGIPARFSGSGSGTDFTLTISSLEPE DFAVYYCQQWNSNPPTFGQGTKVEIKRTVAAPSVFIFPP SDEQLKSGTASVVCLLNNFYPREAKVQWKVDNALQS GNSQESVTEQDSKDSTYSLSSTLTLSKADYEKHKVYAC EVTHQGLSSPVTKSFNRGEC | 31 |

**Table 6. CDRs of chimeric antibodies**

| No. | VH CDR1 SEQ ID NO: | VH CDR2 SEQ ID NO: | VH CDR3 SEQ ID NO: | VL CDR1 SEQ ID NO: | VL CDR2 SEQ ID NO: | VL CDR3 SEQ ID NO: |
|---|---|---|---|---|---|---|
| M1 | 6 | 7 | 8 | 9 | 14 | 19 |
| M2 | 6 | 7 | 8 | 10 | 15 | 20 |
| M3 | 6 | 7 | 8 | 11 | 16 | 21 |
| M4 | 6 | 7 | 8 | 12 | 17 | 22 |
| M5 | 6 | 7 | 8 | 13 | 18 | 23 |

### Example 3. Affinity and Specificity of Chimeric Antibodies

### 1. Verification of affinity of chimeric antibodies-1 Biacore

BiaCore T200 (GE Healthcare) was used for detection: a Protein A chip (GE Healthcare, Cas#29127556) was used for detection, the antibody was diluted to 5 µg/mL using 1× HBS-EP+ (preparation of 1× HBS-EP+: adding 50 mL of HBS-EP+ (10×) (GE Healthcare, cat#BR-1006-69) to 450 mL of ultrapure water and mixing) and then passed through the experimental flow channels (Fc2, Fc4) at a flow rate of 10 µL/min, and the capturing lasted for 20 s so that the capture amount was about 235 RU; then the flow rate was adjusted to 30 µL/min, hB7-H3-His dilutions at different concentrations (0 nM, 3.125 nM, 6.25 nM, 12.5 nM, 25 nM and 50 nM, diluted with 1× HBS-EP+) were fed in sequence and each simultaneously passed through the surfaces of the experimental flow channels (Fc2, Fc4) and reference flow channels (Fc1, Fc3), the binding time was 180 s, and the dissociation time was 600 s; finally the chip was regenerated with Glycine pH 1.5 (GE Healthcare, cat#BR100354) for 60 s, and then the next cycle was performed. Experiment results were analyzed using data analysis software Evaluation Software 3.1. The sensing signals acquired from the experiment flow channels for the samples were subjected to double subtraction (minus values of reference flow channels and blank control), and the fitting was performed using a "1:1" kinetic model to obtain the kinetic parameters (ka: association rate; kd: dissociation rate; kD: equilibrium dissociation constant) for the binding of each sample to hB7-H3-His.

**Table 8. Association rates, dissociation rates and equilibrium dissociation constants for the binding of chimeric antibodies to hB7-H3-His**

| Antibody | ka (1/Ms) | kd (1/s) | KD (M) |
|---|---|---|---|
| M1 | 7.504E+5 | 2.794E-4 | 3.723E-10 |
| M2 | 1.282E+5 | 0.04906 | 3.828E-7 |
| M30-H1-L4 | 2.437E+5 | 2.403E-4 | 9.860E-10 |

The results are shown in Table 8. Antibodies M1 and M2 were both able to bind to hB7-H3-His, and antibody M1 bound to hB7-H3-His with a higher affinity.

### 2. Verification of affinity of chimeric antibodies-2 flow cytometry

MDA-MB-468 cells in logarithmic phase were collected and centrifuged at 1500 rpm for 5 min, and the supernatant was removed. The cells were resuspended in 1× PBS and centrifuged at 1500 rpm for 5 min, and the supernatant was removed, and then the procedure was repeated once. MDA-MB-468 cells were added to a V-bottom 96-well plate at 50 µL/well (200,000 cells) and centrifuged at 1500 rpm for 5 min, and PBS was discarded; the chimeric antibody diluted in gradient (initial concentration of 100 nM, 3-fold gradient dilution, 1× PBS as the vehicle) was added at 100 µL/well; the cells were resuspended, and incubated on ice for 1 h; after the incubation was completed, the cells were centrifuged at 2000 rpm for 5 min, and the supernatant was removed; the cells were resuspended in 1× PBS added at 200 µL/well and centrifuged again and the supernatant was removed, and the procedure was repeated twice; a PE-labeled goat anti-human IgG Fc secondary antibody (Invitrogen, cat# 12-4998-82, diluted with 1× PBS at 1:500) was added at 100 µL/well; the cells were resuspended, and incubated for 30 min on ice in the absence of light; after the incubation was completed, the cells were centrifuged at 2000 rpm for 5 min, and the supernatant was removed; the cells were resuspended in 1× PBS added at 200 µL/well and centrifuged again and the supernatant was removed, and the procedure was repeated twice; 1× PBS was then added at 200 µL/well to resuspend the cells; the detection was performed with a cytoflex flow cytometer (Beckman).

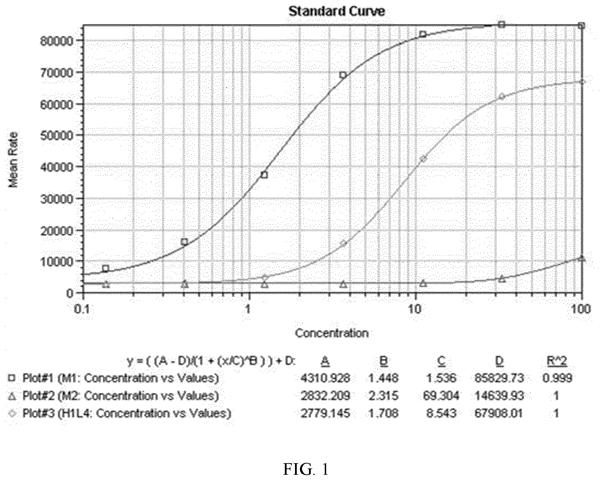

The detection results are shown in FIG. 1.

### 3. Verification of specificity of chimeric antibody-flow cytometry

Cells (Lag3-CHO cells, VISTA-CHO cells, Tim3-CHO cells or Raji cells) in logarithmic phase were collected and centrifuged at 1500 rpm for 5 min, and the supernatant was removed. The cells were resuspended in 1× PBS and centrifuged at 1500 rpm for 5 min, and the supernatant was removed, and then the procedure was repeated once. The cells were added to a V-bottom 96-well plate at 50 µL/well (500,000 cells) and centrifuged at 1500 rpm for 5 min, and PBS was discarded; the antibody M1 (at a concentration of 100 nM) was added at 100 µL/well; the cells were resuspended, and incubated on ice for 1 h; after the incubation was completed, the cells were centrifuged at 2000 rpm for 5 min, and the supernatant was removed; the cells were resuspended in 1× PBS added at 200 µL/well and centrifuged again and the supernatant was removed, and the procedure was repeated twice; a PE-labeled goat anti-human IgG Fc secondary antibody (Invitrogen, cat# 12-4998-82, diluted with 1× PBS at 1:500) was added at 100 µL/well; the cells were resuspended, and incubated for 30 min on ice in the absence of light; after the incubation was completed, the cells were centrifuged at 2000 rpm for 5 min, and the supernatant was removed; the cells were resuspended in 1× PBS added at 200 µL/well and centrifuged again and the supernatant was removed, and the procedure was repeated twice; 1× PBS was then added at 200 µL/well to resuspend the cells; the detection was performed with a cytoflex flow cytometer (Beckman).

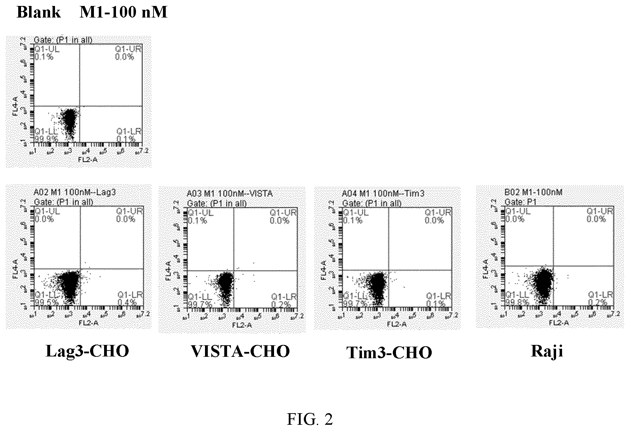

The results are shown in FIG. 2. Antibody M1 did not bind to Lag3-CHO cells, VISTA-CHO cells, Tim3-CHO cells or Raji cells.

### Example 4. Humanized Antibodies

The amino acid sequences of the VHs and VLs of the humanized antibodies are shown in Table 9. The assembly scheme of the humanized antibodies is shown in Table 10, the VH and CH constitute the heavy chain of the antibody, and the VL and CL constitute the light chain of the antibody; the antibodies are indicated by "antibody" + antibody number, e.g., the heavy chain of antibody V3 consists of the VH set forth in SED ID NO: 36 and the CH set forth in SED ID NO: 32, and the light chain of antibody V3 consists of the VL set forth in SED ID NO: 40 and the CL set forth in SED ID NO: 34. The heavy chain of the control antibody M30-H1-L4 is set forth in SEQ ID NO: 30 and its light chain is set forth in SEQ ID NO: 31. The heavy chain of the control antibody Trop2 is set forth in SEQ ID NO: 42 and its light chain is set forth in SEQ ID NO: 43 (see Table 11).

**Table 9. Amino acid sequences of humanized VHs and VLs**

| No. | Type | Amino acid sequence | SEQ ID NO: |
|---|---|---|---|
| hMH-1 | VH | EVQLVQSGAEVKKSGESLKISCKASGYTFTDYDINWVRQMPGKG LEWMGWIFPGDDTTKYNEKFKGQVTISADKSTNTAYLQWSSLK ASDTAMYYCARSPSFDYWGQGTLVTVSS | 35 |
| hMH-2 | | EVQLVQSGAEVKKSGESLKISCKASGYTFTDYDINWVRQMPGKG LEWIGWIFPGDDTTKYNEKFKGQVTLSADKSTNTAYMQWSSLK ASDTAMYYCARSPSFDYWGQGTLVTVSS | 36 |
| hMH-3 | | EVQLVESGGGLVQPGGSLRLSCAASGYTFTDYDINWVRQAPGKG LEWVGWIFPGDDTTKYNEKFKGRFTISADKSKNTAYLQMNSLR AEDTAVYYCARSPSFDYWGQGTLVTVSS | 37 |
| hMH-4 | | QVQLVQSGAEVKKPGASVKVSCKASGYTFTDYDINWVRQAPGQ RLEWIGWIFPGDDTTKYNEKFKGRATLTADKSASTAYMELSSLR SEDTAVYYCARSPSFDYWGQGTLVTVSS | 38 |
| hML-1 | VL | QIVLTQSPGTLSLSPGERATLSCSASSTIGFMYWYQQKPGQAPRR WIYDTSKLASGVPDRFSVSGSGTDYTLTISRLEPEDFAVYYCHQRS SYPTFGQGTKVEIK | 39 |
| hML-2 | | QIVLTQSPGTLSLSPGERATLSCSASSTIGFMYWYQQKPGQAPRR WIYDTSKLASGVPDRFSGSGSGTDYTLTISRLEPEDFAVYYCHQRS SYPTFGQGTKVEIK | 40 |
| hML-3 | | QIVLTQSPSSLSASVGDRVTITCSASSTIGFMYWYQQKPGKAPKR WIYDTSKLASGVPSRFSVSGSGTDYTLTISSLQPEDFATYYCHQRS SYPTFGQGTKVEIK | 41 |

| | | | |
|---|---|---|---|
| Note: CDRs (Kabat numbering) are bolded and underlined | | | |

**Table 10. Number and assembly scheme of humanized antibodies**

| Antibody No. | VH SEQ ID NO: | CH SEQ ID NO: | VL SEQ ID NO: | CL SEQ ID NO: |
|---|---|---|---|---|
| V1 | 35 | 32 | 39 | 34 |
| V2 | 36 | 32 | 39 | 34 |
| V3 | 36 | 32 | 40 | 34 |
| V4 | 37 | 32 | 41 | 34 |
| V5 | 38 | 32 | 41 | 34 |

**Table 11. Amino acid sequence of control antibody Trop2**

| Antibody No. | Type | Amino acid sequence | SEQ ID NO: |
|---|---|---|---|
| Trop2 | Heavy chain | QVQLQQSGSELKKPGASVKVSCKASGYTFTNYGMN WVKQAPGQGLKWMGWINTYTGEPTYTDDFKGRFAF SLDTSVSTAYLQISSLKADDTAVYFCARGGFGSSYWYF DVWGQGTLVTVSSASTKGPSVFPLAPSSKSTSGGTAA LGCLVKDYFPEPVTVSWNSGALTSGVHTFPAVLQSSG LYSLSSVVTVPSSSLGTQTYICNVNHKPSNTKVDKKV EPKSCDKTHTCPPCPAPELLGGPSVFLFPPKPKDTLMIS RTPEVTCVVVDVSHEDPEVKFNWYVDGVEVHNAKT KPREEQYNSTYRVVSVLTVLHQDWLNGKEYKCKVSN KALPAPIEKTISKAKGQPREPQVYTLPPSRDELTKNQV SLTCLVKGFYPSDIAVEWESNGQPENNYKTTPPVLDSD GSFFLYSKLTVDKSRWQQGNVFSCSVMHEALHNHYT QKSLSLSPGK | 42 |
| Trop2 | Light chain | DIQLTQSPSSLSASVGDRVSITCKASQDVSIAVAWYQQ KPGKAPKLLIYSASYRYTGVPDRFSGSGSGTDFTLTISS LQPEDFAVYYCQQHYITPLTFGAGTKVEIKRTVAAPSV FIFPPSDEQLKSGTASVVCLLNNFYPREAKVQWKVDN ALQSGNSQESVTEQDSKDSTYSLSSTLTLSKADYEKH KVYACEVTHQGLSSPVTKSFNRGEC | 43 |

Gene sequences of the heavy chain and the light chain of the antibody were cloned into an expression vector to obtain a recombinant expression vector, which was then transiently transfected into HEK293F cells. After culturing and Protein A affinity chromatography purification, the humanized antibody was obtained and its sequence was consistent with the expected sequence as determined by sequencing.

### Example 5. Affinity, Internalization Capacity and Specificity of Humanized Antibodies

### 1. Verification of affinity of humanized antibodies-1 ELISA

The day before the experiment, the target antigen hB7-H3-Fc was diluted to 2 µg/mL with 1× PBS and added to an ELISA plate (96-well plate) at 100 µL/well, and the wells were coated overnight at 4 °C; the next day, the coating solution was removed by spin-drying, the wells were washed with the washing solution PBST twice and patted dry, the blocking solution (PBST containing 1% BSA) was added at 200 µL/well, and the wells were blocked for 2 h at 37 °C; the blocking solution was removed by spin-drying, the wells were washed with the washing solution PBST twice and patted dry, the humanized antibody diluted in gradient (initial concentration of 300 nM, 3-fold gradient dilution, 1× PBS as the vehicle) was added at 100 µL/well, and the mixture was reacted at 37 °C for 1 h; the humanized antibody was removed by spin-drying, the wells were washed with the washing solution PBST 5 times and patted dry, an HRP-labeled goat anti-human Kappa light chain secondary antibody (Invitrogen, cat#A18853, diluted with 1× PBS at 1:2000) was added at 100 µL/well, and the mixture was reacted at 37 °C for 1 h; the secondary antibody was removed by spin-drying, the wells were washed with the washing solution PBST 8 times and patted dry, a TMB substrate solution was added at 100 µL/well, and the mixture was reacted at 37 °C for 5-10 min; then an ELISA stop solution was added at 100 µL/well, and the absorbance at 450 nm wavelength was read using a microplate reader within 15 min.

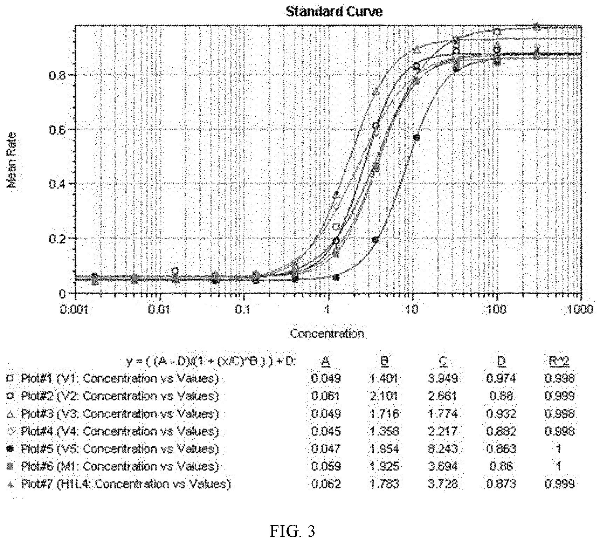

The results are shown in FIG. 3.

### 2. Verification of affinity of humanized antibodies-2 flow cytometry

MDA-MB-468 cells in logarithmic phase were collected and centrifuged at 1500 rpm for 5 min, and the supernatant was removed. The cells were resuspended in 1× PBS and centrifuged at 1500 rpm for 5 min, and the supernatant was removed, and then the procedure was repeated once. MDA-MB-468 cells were added to a V-bottom 96-well plate at 50 µL/well (200,000 cells) and centrifuged at 1500 rpm for 5 min, and PBS was discarded; the humanized antibody diluted in gradient (initial concentration of 100 nM, 3-fold gradient dilution, 1× PBS as the vehicle) was added at 100 µL/well; the cells were resuspended, and incubated on ice for 1 h; after the incubation was completed, the cells were centrifuged at 2000 rpm for 5 min, and the supernatant was removed; the cells were resuspended in 1× PBS added at 200 µL/well and centrifuged again and the supernatant was removed, and the procedure was repeated twice; a PE-labeled goat anti-human IgG Fc secondary antibody (Invitrogen, cat# 12-4998-82, diluted with 1× PBS at 1:500) was added at 100 µL/well; the cells were resuspended, and incubated for 30 min on ice in the absence of light; after the incubation was completed, the cells were centrifuged at 2000 rpm for 5 min, and the supernatant was removed; the cells were resuspended in 1× PBS added at 200 µL/well and centrifuged again and the supernatant was removed, and the procedure was repeated twice; 1× PBS was then added at 200 µL/well to resuspend the cells; the detection was performed with a cytoflex flow cytometer (Beckman).

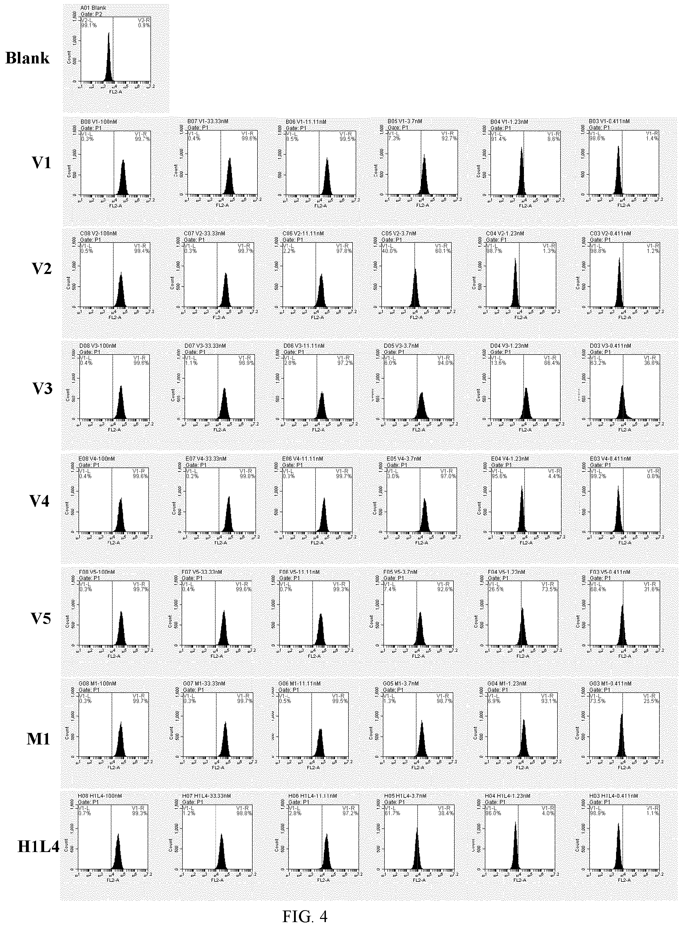

The results are shown in FIG. 4.

### 3. Detection of internalization of humanized antibodies by flow cytometry

30 µg/mL of humanized antibody was prepared by using a cell washing solution (PBS containing 2% FBS) pre-cooled on ice and well mixed with cells (MDA-MB-468 cells or N87 cells, 500,000 cells) according to a volume ratio of 1:1 (the final volume is 100 µL), and the mixture was incubated in an ice bath for 1 h; the mixture was then centrifuged at 1200 r/min and 4 °C for 5 min, and the supernatant was discarded; the cells were resuspended in the cell washing solution pre-cooled on ice and centrifuged at 1200 r/min and 4 °C for 5 min and the supernatant was removed, and the procedure was repeated twice; the cells were resuspended in 200 µL of cell washing solution pre-cooled on ice, incubated at 37 °C for 2 h to internalize the antibody bound to the cell surface and then transferred to an ice bath, and the cell washing solution pre-cooled on ice was added to terminate the internalization; the cells were centrifuged at 1200 r/min and 4 °C for 5 min, the supernatant was discarded, 100 µL of a PE-labeled goat anti-human IgG Fc secondary antibody diluent (Invitrogen, cat#12-4998-82, prepared with the cell washing solution pre-cooled on ice and a PE-labeled goat anti-human IgG Fc secondary antibody (500:1)) was added; the cells were resuspended, and incubated at 4 °C for 30 min in the absence of light and centrifuged at 1200 r/min and 4 °C for 5 min, and the supernatant was discarded; the cells were resuspended in the cell washing solution pre-cooled on ice and centrifuged at 1200 r/min and 4 °C for 5 min and the supernatant was discarded, and the cells were resuspended in 1× PBS pre-cooled on ice and centrifuged at 1200 r/min and 4 °C for 5 min and the supernatant was discarded; 200 µL of 1× PBS pre-cooled on ice was added to resuspend the cells, and the mean fluorescence intensity was detected with a flow cytometer.

**Table 12. Internalization capacity of humanized antibodies**

| Cell | MDA-MB-468 cells | | | N87 cells | | |
|---|---|---|---|---|---|---|
| Antibody | Trop2 | V1 | V3 | Trop2 | V1 | V3 |
| Fluorescence intensity after endocytosis | 69153.17 | 47449.34 | 36060.02 | 174366.53 | 222436.23 | 193742.47 |
| Fluorescence intensity of control group (before endocytosis) | 165668.35 | 53567.67 | 47757.42 | 334120.58 | 236990.73 | 246249.85 |
| Percentage of endocytosis | 58.26% | 11.42% | 24.49% | 47.81% | 6.14% | 21.32% |

### 4. Verification of specificity of humanized antibodies-flow cytometry

Cells (VISTA-CHO cells, Tim3-CHO cells or Lag3-CHO cells) in logarithmic phase were collected and centrifuged at 1500 rpm for 5 min, and the supernatant was removed. The cells were resuspended in 1× PBS and centrifuged at 1500 rpm for 5 min, and the supernatant was removed, and then the procedure was repeated once. The cells were added to a V-bottom 96-well plate at 50 µL/well (500,000 cells) and centrifuged at 1500 rpm for 5 min, and PBS was discarded; the humanized antibody (at a concentration of 100 nM, 1× PBS as the vehicle) was added at 100 µL/well; the cells were resuspended, and incubated on ice for 1 h; after the incubation was completed, the cells were centrifuged at 2000 rpm for 5 min, and the supernatant was removed; the cells were resuspended in 1× PBS added at 200 µL/well and centrifuged again and the supernatant was removed, and the procedure was repeated twice; a PE-labeled goat anti-human IgG Fc secondary antibody (Invitrogen, cat#12-4998-82, diluted with 1× PBS at 1:500) was added at 100 µL/well; the cells were resuspended, and incubated for 30 min on ice in the absence of light; after the incubation was completed, the cells were centrifuged at 2000 rpm for 5 min, and the supernatant was removed; the cells were resuspended in 1× PBS added at 200 µL/well and centrifuged again and the supernatant was removed, and the procedure was repeated twice; 1× PBS was then added at 200 µL/well to resuspend the cells; the detection was performed with a cytoflex flow cytometer (Beckman).

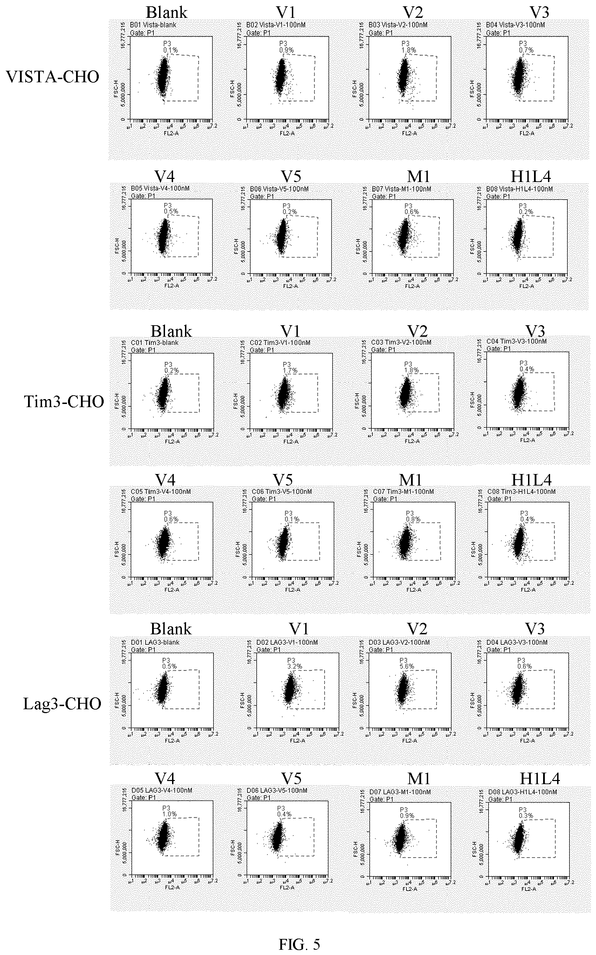

The detection results are shown in FIG. 5. None of the antibodies V1-V5 bound to VISTA-CHO cells, Tim3-CHO cells or Lag3-CHO cells.

### 5. Species specificity of humanized antibody

The day before the experiment, the target antigens cynomolgus monkey B7-H3 (novoprotein CA61), mouse B7-H3 (SinoBiological, 50973-M08H) and hB7-H3-his were each diluted to 2 µg/mL with 1 × PBS and added to a ELISA plate (96-well plate) at 100 µL/well (two rows for each antigen), and the wells were coated overnight at 4 °C. The next day, the coating solution was removed by spin-drying, the wells were washed with the washing solution PBST twice and patted dry, the blocking solution was added at 200 µL/well, and the wells were blocked for 2 h at 37 °C; the blocking solution was removed by spin-drying, the wells were washed with the washing solution PBST twice and patted dry, the antibody V3 diluted in gradient (initial concentration of 2 µg/mL, 2-fold gradient dilution) was added at 100 µL/well, and the mixture was reacted at 37 °C for 2 h; the antibody V3 was removed by spin-drying, the wells were washed with the washing solution PBST 5 times and patted dry, an anti-human kappa light Chains HRP (Sigma, A7164, diluted with 1× PBS at 1:10000) was added at 100 µL/well, and the mixture was reacted at 37 °C for 1 h; the secondary antibody was removed by spin-drying, the wells were washed with the washing solution PBST 8 times and patted dry, a TMB substrate solution was added at 100 µL/well, and the mixture was reacted at 37 °C for 10 min; then an 0.1 M H₂SO₄ stop solution was added at 100 µL/well, and the absorbance at 450 nm wavelength was read using a microplate reader within 15 min.

**Table 13. Affinity of antibody V3 for binding to B7-H3**

| Antigen | EC₅₀ (µg/mL) |
|---|---|
| Mouse B7-H3 | NA |
| Cynomolgus monkey B7-H3 | 0.042 |
| hB7-H3-his | 0.042 |

As can be seen from Table 13, antibody V3 bound to human and cynomolgus monkey B7-H3 but did not bind to mouse B7-H3.

## Claims

1. An antibody or an antigen-binding fragment, wherein the antibody or the antigen-binding fragment specifically binds to B7-H3 and comprises one or more of amino acid sequences of (a)-(f):
(a) a VH CDR1 comprising an amino acid sequence set forth in SEQ ID NO: 6;
(b) a VH CDR2 comprising an amino acid sequence set forth in SEQ ID NO: 7;
(c) a VH CDR3 comprising an amino acid sequence set forth in SEQ ID NO: 8;
(d) a VL CDR1 comprising an amino acid sequence set forth in any one of SEQ ID NOs: 9-13;
(e) a VL CDR2 comprising an amino acid sequence set forth in any one of SEQ ID NOs: 14-18; and
(f) a VL CDR3 comprising an amino acid sequence set forth in any one of SEQ ID NOs: 19-23.

2. The antibody or the antigen-binding fragment according to claim 1, wherein the antibody or the antigen-binding fragmen comprises a VH CDR1 set forth in SEQ ID NO: 6, a VH CDR2 set forth in SEQ ID NO: 7 and a VH CDR3 set forth in SEQ ID NO: 8.

3. The antibody or the antigen-binding fragment according to claim 1 or 2, wherein the antibody or the antigen-binding fragment comprises a VL CDR1 set forth in any one of SEQ ID NOs: 9-13, a VL CDR2 set forth in any one of SEQ ID NOs: 14-18 and a VL CDR3 set forth in any one of SEQ ID NOs: 19-23.

4. The antibody or the antigen-binding fragment according to any one of claims 1-3, wherein the antibody or the antigen-binding fragment comprises a VH CDR1 set forth in SEQ ID NO: 6, a VH CDR2 set forth in SEQ ID NO: 7, a VH CDR3 set forth in SEQ ID NO: 8, a VL CDR1 set forth in SEQ ID NO: 9, a VL CDR2 set forth in SEQ ID NO: 14 and a VL CDR3 set forth in SEQ ID NO: 19; or
the antibody or the antigen-binding fragment comprises a VH CDR1 set forth in SEQ ID NO: 6, a VH CDR2 set forth in SEQ ID NO: 7, a VH CDR3 set forth in SEQ ID NO: 8, a VL CDR1 set forth in SEQ ID NO: 10, a VL CDR2 set forth in SEQ ID NO: 15 and a VL CDR3 set forth in SEQ ID NO: 20; or
the antibody or the antigen-binding fragment comprises a VH CDR1 set forth in SEQ ID NO: 6, a VH CDR2 set forth in SEQ ID NO: 7, a VH CDR3 set forth in SEQ ID NO: 8, a VL CDR1 set forth in SEQ ID NO: 11, a VL CDR2 set forth in SEQ ID NO: 16 and a VL CDR3 set forth in SEQ ID NO: 21; or
the antibody or the antigen-binding fragment comprises a VH CDR1 set forth in SEQ ID NO: 6, a VH CDR2 set forth in SEQ ID NO: 7, a VH CDR3 set forth in SEQ ID NO: 8, a VL CDR1 set forth in SEQ ID NO: 12, a VL CDR2 set forth in SEQ ID NO: 17 and a VL CDR3 set forth in SEQ ID NO: 22; or
the antibody or the antigen-binding fragment comprises a VH CDR1 set forth in SEQ ID NO: 6, a VH CDR2 set forth in SEQ ID NO: 7, a VH CDR3 set forth in SEQ ID NO: 8, a VL CDR1 set forth in SEQ ID NO: 13, a VL CDR2 set forth in SEQ ID NO: 18 and a VL CDR3 set forth in SEQ ID NO: 23.

5. The antibody or the antigen-binding fragment according to any one of claims 1-4, wherein a heavy chain variable region of the antibody or the antigen-binding fragment comprises an amino acid sequence set forth in any one of SEQ ID NOs: 24 and 35-38, or an amino acid sequence having at least 80% or 90% identity compared with the amino acid sequence set forth in any one of SEQ ID NOs: 24 and 35-38, or an amino acid sequence having one or more conservative amino acid substitutions compared with the amino acid sequence set forth in any one of SEQ ID NOs: 24 and 35-38; and/or
a light chain variable region of the antibody or the antigen-binding fragment comprises an amino acid sequence set forth in any one of SEQ ID NOs: 25-29 and 39-41, or an amino acid sequence having at least 80% or 90% identity compared with the amino acid sequence set forth in any one of SEQ ID NOs: 25-29 and 39-41, or an amino acid sequence having one or more conservative amino acid substitutions compared with the amino acid sequence set forth in any one of SEQ ID NOs: 25-29 and 39-41.

6. An antibody or an antigen-binding fragment, wherein a heavy chain variable region of the antibody or the antigen-binding fragment comprises an amino acid sequence set forth in SEQ ID NO: 24, and a light chain variable region of the antibody or the antigen-binding fragment comprises an amino acid sequence set forth in SEQ ID NO: 25; or
a heavy chain variable region of the antibody or the antigen-binding fragment comprises an amino acid sequence set forth in SEQ ID NO: 24, and a light chain variable region of the antibody or the antigen-binding fragment comprises an amino acid sequence set forth in SEQ ID NO: 26; or
a heavy chain variable region of the antibody or the antigen-binding fragment comprises an amino acid sequence set forth in SEQ ID NO: 24, and a light chain variable region of the antibody or the antigen-binding fragment comprises an amino acid sequence set forth in SEQ ID NO: 27; or
a heavy chain variable region of the antibody or the antigen-binding fragment comprises an amino acid sequence set forth in SEQ ID NO: 24, and a light chain variable region of the antibody or the antigen-binding fragment comprises an amino acid sequence set forth in SEQ ID NO: 28; or
a heavy chain variable region of the antibody or the antigen-binding fragment comprises an amino acid sequence set forth in SEQ ID NO: 24, and a light chain variable region of the antibody or the antigen-binding fragment comprises an amino acid sequence set forth in SEQ ID NO: 29;
or a heavy chain variable region of the antibody or the antigen-binding fragment comprises an amino acid sequence set forth in SEQ ID NO: 35, and a light chain variable region of the antibody or the antigen-binding fragment comprises an amino acid sequence set forth in SEQ ID NO: 39; or
a heavy chain variable region of the antibody or the antigen-binding fragment comprises an amino acid sequence set forth in SEQ ID NO: 36, and a light chain variable region of the antibody or the antigen-binding fragment comprises an amino acid sequence set forth in SEQ ID NO: 39; or
a heavy chain variable region of the antibody or the antigen-binding fragment comprises an amino acid sequence set forth in SEQ ID NO: 36, and a light chain variable region of the antibody or the antigen-binding fragment comprises an amino acid sequence set forth in SEQ ID NO: 40; or
a heavy chain variable region of the antibody or the antigen-binding fragment comprises an amino acid sequence set forth in SEQ ID NO: 37, and a light chain variable region of the antibody or the antigen-binding fragment comprises an amino acid sequence set forth in SEQ ID NO: 41; or
a heavy chain variable region of the antibody or the antigen-binding fragment comprises an amino acid sequence set forth in SEQ ID NO: 38, and a light chain variable region of the antibody or the antigen-binding fragment comprises an amino acid sequence set forth in SEQ ID NO: 41.

7. The antibody or the antigen-binding fragment according to any one of claims 1-6, wherein the antibody or the antigen-binding fragment is of IgG isotype; or the antibody or the antigen-binding fragment is of IgG1 or IgG4 isotype.

8. The antibody or the antigen-binding fragment according to any one of claims 1-7, wherein the antibody or the antigen-binding fragment further comprises a heavy chain constant region and/or a light chain constant region, wherein the heavy chain constant region comprises an amino acid sequence set forth in SEQ ID NO: 32 or 33, or an amino acid sequence having at least 80% or 90% identity compared with the amino acid sequence set forth in SEQ ID NO: 32 or 33, or an amino acid sequence having one or more conservative amino acid substitutions compared with the amino acid sequence set forth in SEQ ID NO: 32 or 33; and/or
the light chain constant region comprises an amino acid sequence set forth in SEQ ID NO: 34, or an amino acid sequence having at least 80% or at least 90% identity compared with the amino acid sequence set forth in SEQ ID NO: 34, or an amino acid sequence having one or more conservative amino acid substitutions compared with the amino acid sequence set forth in SEQ ID NO: 34.

9. An antibody, wherein a heavy chain of the antibody comprises a heavy chain variable region having an amino acid sequence set forth in SEQ ID NO: 24 and a heavy chain constant region having an amino acid sequence set forth in SEQ ID NO: 32; a light chain of the antibody comprises a light chain variable region having an amino acid sequence set forth in SEQ ID NO: 25 and a light chain constant region having an amino acid sequence set forth in SEQ ID NO: 34; or
a heavy chain of the antibody comprises a heavy chain variable region having an amino acid sequence set forth in SEQ ID NO: 24 and a heavy chain constant region having an amino acid sequence set forth in SEQ ID NO: 32; a light chain of the antibody comprises a light chain variable region having an amino acid sequence set forth in SEQ ID NO: 26 and a light chain constant region having an amino acid sequence set forth in SEQ ID NO: 34; or
a heavy chain of the antibody comprises a heavy chain variable region having an amino acid sequence set forth in SEQ ID NO: 24 and a heavy chain constant region having an amino acid sequence set forth in SEQ ID NO: 32; a light chain of the antibody comprises a light chain variable region having an amino acid sequence set forth in SEQ ID NO: 27 and a light chain constant region having an amino acid sequence set forth in SEQ ID NO: 34; or
a heavy chain of the antibody comprises a heavy chain variable region having an amino acid sequence set forth in SEQ ID NO: 24 and a heavy chain constant region having an amino acid sequence set forth in SEQ ID NO: 32; a light chain of the antibody comprises a light chain variable region having an amino acid sequence set forth in SEQ ID NO: 28 and a light chain constant region having an amino acid sequence set forth in SEQ ID NO: 34; or
a heavy chain of the antibody comprises a heavy chain variable region having an amino acid sequence set forth in SEQ ID NO: 24 and a heavy chain constant region having an amino acid sequence set forth in SEQ ID NO: 32; a light chain of the antibody comprises a light chain variable region having an amino acid sequence set forth in SEQ ID NO: 29 and a light chain constant region having an amino acid sequence set forth in SEQ ID NO: 34; or
a heavy chain of the antibody comprises a heavy chain variable region having an amino acid sequence set forth in SEQ ID NO: 35 and a heavy chain constant region having an amino acid sequence set forth in SEQ ID NO: 32; a light chain of the antibody comprises a light chain variable region having an amino acid sequence set forth in SEQ ID NO: 39 and a light chain constant region having an amino acid sequence set forth in SEQ ID NO: 34; or
a heavy chain of the antibody comprises a heavy chain variable region having an amino acid sequence set forth in SEQ ID NO: 36 and a heavy chain constant region having an amino acid sequence set forth in SEQ ID NO: 32; a light chain of the antibody comprises a light chain variable region having an amino acid sequence set forth in SEQ ID NO: 39 and a light chain constant region having an amino acid sequence set forth in SEQ ID NO: 34; or
a heavy chain of the antibody comprises a heavy chain variable region having an amino acid sequence set forth in SEQ ID NO: 36 and a heavy chain constant region having an amino acid sequence set forth in SEQ ID NO: 32; a light chain of the antibody comprises a light chain variable region having an amino acid sequence set forth in SEQ ID NO: 40 and a light chain constant region having an amino acid sequence set forth in SEQ ID NO: 34; or
a heavy chain of the antibody comprises a heavy chain variable region having an amino acid sequence set forth in SEQ ID NO: 37 and a heavy chain constant region having an amino acid sequence set forth in SEQ ID NO: 32; a light chain of the antibody comprises a light chain variable region having an amino acid sequence set forth in SEQ ID NO: 41 and a light chain constant region having an amino acid sequence set forth in SEQ ID NO: 34; or
a heavy chain of the antibody comprises a heavy chain variable region having an amino acid sequence set forth in SEQ ID NO: 38 and a heavy chain constant region having an amino acid sequence set forth in SEQ ID NO: 32; a light chain of the antibody comprises a light chain variable region having an amino acid sequence set forth in SEQ ID NO: 41 and a light chain constant region having an amino acid sequence set forth in SEQ ID NO: 34.

10. A biomaterial, being:
a polynucleotide, wherein the polynucleotide encodes the antibody or the antigen-binding fragment according to any one of claims 1-8 or part or all of the antibody according to claim 9; or
an expression vector, wherein the expression vector comprises a polynucleotide encoding the antibody or the antigen-binding fragment according to any one of claims 1-8 or part or all of the antibody according to claim 9; or
a cell, wherein the cell comprises a polynucleotide encoding the antibody or the antigen-binding fragment according to any one of claims 1-8 or part or all of the antibody according to claim 9.

11. A pharmaceutical composition, wherein the pharmaceutical composition comprises the antibody or the antigen-binding fragment according to any one of claims 1-8 or part or all of the antibody according to claim 9, or the biomaterial according to claim 10, and a pharmaceutically acceptable carrier.

12. Use of the antibody or the antigen-binding fragment according to any one of claims 1-8 or part or all of the antibody according to claim 9, or the biomaterial according to claim 10, or the pharmaceutical composition according to claim 11 in the manufacture of a drug for diagnosing and/or treating a disease;
or the disease is selected from a tumor, a respiratory disease, a skin and musculoskeletal disease, a genitourinary disease, a nervous system disease and a digestive system disease; or the tumor is a benign tumor or cancer; or the tumor is a hematological tumor or a solid tumor; or the tumor is a tumor positive for B7-H3 expression; or the tumor is selected from melanoma, lung cancer such as non-small cell lung cancer, colorectal cancer, head and neck cancer, kidney cancer, prostate cancer such as castration-resistant prostate cancer, breast cancer, gastric cancer, liver cancer such as hepatocellular carcinoma, cervical cancer, ovarian cancer such as ovarian epithelial carcinoma, glioma such as childhood brain stem glioma, pancreatic cancer such as pancreatic ductal carcinoma, leukemia, mesothelioma, squamous cell carcinoma, neuroblastoma, desmoplastic small round cell tumor, medulloblastoma, meningioma, peritoneal malignancy, sarcoma, brain cancer, central nervous system tumor and metastatic brain tumor.
